# EUROPEAN PATENT SPECIFICATION

(11) **EP 0 480 676 B1**
(45) Date of publication and mention of the grant of the patent: **10.04.1996**
(21) Application number: 91309212.8
(22) Date of filing: 08.10.1991
(51) Int. Cl.: C12N 9/16, C12Q 1/44, C12N 1/20

(54) **Acyl carnitine esterase, production thereof and method for analyzing acyl carnitines**
Acyl-carnitin-Esterase, Verfahren zu ihrer Herstellung und Verfahren zur Analyse von Acyl-carnitinen
Acyl-carnitine-estérase, procédé de préparation et procédé d'analyse d'acyl-carnitines

(30) Priority: 09.10.1990 JP 270784/90
(43) Date of publication of application: 15.04.1992
(73) Proprietor: ASAHI KASEI KOGYO KABUSHIKI KAISHA, Osaka (JP)
(72) Inventor: Takahashi, Mamoru, Sunto-gun, Shizuoka (JP); Ueda, Shigeru, Tagata-gun, Shizuoka (JP)
(74) Representative: Hayes, Adrian Chetwynd (GB)

(56) References cited:
- ARCHIVES OF BIOCHEMISTRY & BIOPHYSICS, vol. 277, no. 1, 15 February 1990, New York, NY (US); K. BRUNS et al., pp. 1-7
- BIOCHEMICAL JOURNAL, vol. 152, November 1975, London (GB); N.D. COSTA et al., pp. 161-166
- EUROPEAN JOURNAL OF BIOCHEMISTRY, vol. 6, 1968, Berlin (DE); H. AURICH et al., pp. 196-201
- WPIL, Week 8702, Derwent Publications Ltd., London (GB); AN 87-012544
- MEDLINE DATABASE; J. KERNER et al., AN 84-077694

## Description

This invention relates to an acyl-carnitine esterase which is useful for analyzing acyl-L-carnitines in, for example, a biochemical clinic inspection or a screening inspection of food products; a process for the production thereof; and a use thereof for analyzing acyl-L-carnitines.

L-carnitine, also called vitamin Bγ, is a substance necessary for the transport of fatty acids through mitochondrial membranes in animals. In the cells of animals there are also acylated derivatives of L-carnitine, namely acyl-L-carnitines. Acyl-L-carnitines are excreted from animals and are found in urine. Therefore, quantitative determination of the content of acyl-L-carnitines in, for example, blood is very important in, for example, monitoring the existence of any functional mitochondria disorder in order to check any defect in the intramuscular energy transference in animals.

Analysis of acyl-L-carnitines in substances has previously been realized by hydrolyzing the acyl-L-carnitines with an alkali and quantitatively determining the amount of liberated free L-carnitine. Several reports have proposed conditions for such a hydrolysis, for example, Pearson et al in "Methods in Enzymology", Vol. 14, 621 (1969), Bieber et al in "Methods in Enzymology", Vol. 72, 276 (1981) and Pace et al in "Clin. Chem.", 24, 32 (1978).

These prior techniques suffer from significant shortcomings in that the analysis procedures take a considerable period of time and the use of a highly concentrated alkali solution for the hydrolysis brings about danger in operation and dilutes the sample solution.

In order to overcome such shortcomings, the use of an appropriate enzyme, i.e. an acylcarnitine esterase, may be considered. An acylcarnitine esterase is known which originates from the liver of rats [S. Mahadevan & F. Sauer; "J. Biol. Chem.", 244, 4448-4453 (1069)]. This acylcarnitine esterase has, however, no activity for short chain acyl-L-carnitines, such as acetyl-L-carnitine and propionyl-L-carnitine, existing in human blood. Additionly it has higher Km values for long chain acyl-L-carnitines, for example 3.2 x 10⁻³M for decanoyl-L-carnitine and 5 x 10⁻³M for palmitoyl-L-carnitine, so that a large amount of the esterase is required for complete hydrolysis of long chain acyl-L-carnitines. Moreover, in spite of a large demand for the esterase, only 3.7 Units of the esterase can be collected from 50g of rat liver (about one whole liver of a rat).

Therefore, it has long been desired to find an acylcarnitine esterase which exhibits enough enzymatic activity for short chain acyl-L-carnitines, has lower Km values for substrates and is sufficiently stable, and to develop a reliable and highly sensitive method for the quantitative analysis of acyl-L-carnitines including the short chain acyl-L-carnitines mentioned above that exist in animals.

In these circumstances, the inventors have screened acylcarnitine esterases from a vast number of culture products of bacteria and found that a bacterium of the genus Alcaligenes produces an acyl-L-carnitine esterase exhibiting high activity for the short chain acyl-L-carnitines. This acyl-L-carnitine can be used as a hydolyzing enzyme in a highly sensitive quantitative determination of acyl-L-carnitines contained in a sample.

The present invention provides an acylcarnitine esterase which:
(a) is specific to each of the following substrates:
   acetyl-L-carnitine, propionyl-L-carnitine and palmitoyl-L-carnitine;
(b) catalyzes the hydrolysis of one mole of a said substrate with one mole of water to form one mole of the corresponding acid and one mole of L-carnitine; and
(c) has the following physicochemical properties:
   a) molecular weight of 63,000 ± 7,000 (as determined by gel filtration);
   b) isoelectric point of pH 5.1± 0.5;
   c) Km value for acetyl-L-carnitine of 4 x 10⁻⁵ M, for propionyl-L-carnitine of 3 x 10⁻⁵ M and for palmitoyl-L-carnitine of 2 x 10⁻⁵ M;
   d) optimum pH of around pH 8;
   e) stability at a pH of from 7.5 to 8.5 at 60°C for 30 minutes; and
   f) an optimum temperature of around 70°C at which it exhibits its maximum activity in 100 mM Tris-HCl buffer solution at pH 8.

The present invention also provides a process for the production of an acyl-carnitine esterase as defined above, which comprises cultivating an acylcarnitine esterase-producing bacterium of the genus Alcaligenes in a suitable culture medium and collecting the thereby produced acylcarnitine esterase from the culture product mixture.

The present invention further provides method of assaying acyl-L-carnitines including acetyl-L-carnitine and propionyl-L-carnitine in a sample, which comprises subjecting the sample to enzymatic hydrolysis with an acyl-carnitine esterase as defined above, and then determining the amount of the fatty acids and/or L-carnitine thus formed.

The present invention additionally provides a method of assaying acetyl-L-carnitine and propionyl-L-carnitine in a sample, which comprises:
(a) subjecting part of the sample to enzymatic hydrolysis with an acylcarnitine esterase which has no enzymatic activity for acetyl-L-carnitine and propionyl-L-carnitine but which exhibits substrate-specificity to acyl-L-carnitines of longer chain lengths and which catalyzes the hydrolysis reaction of one mole of acyl-L-carnitine of said longer chain lengths with one mole of water to form one mole of the corresponding fatty acid and one mole of L-carnitine and then determining the amount of the fatty acids and/or L-carnitine thus formed;
(b) subjecting another part of the sample to an assay of acyl-L-carnitines including acetyl-L-carnitine and propionyl-L-carnitine as defined above; and
(c) determining the difference in the analytical values obtained in steps (a) and (b).

The present invention yet further provides a method of assaying acetyl-L-carnitine and propionyl-L-carnitine in a sample, which comprises:
(a₁) subjecting the sample to enzymatic hydrolysis with an acylcarnitine esterase which has no enzymatic activity for acetyl-L-carnitine and propionyl-L-carnitine but which exhibits substrate-specificity to acyl-L-carnitines of longer chain lengths and which catalyzes the hydrolysis reaction of one mole of acyl-L-carnitine of longer chain lengths with one mole of water to form one mole of the corresponding fatty acid and one mole of L-carnitine and then subjecting the resultant reacted sample mixture to the action of an L-carnitine dehydrogenase with a coenzyme A₁ (wherein A₁ is a NAD or thio-NAD group compound) to convert A₁ into its reduced form A₂, and treating the resultant sample mixture to decompose A₂; and
(b₁) subjecting the resultant sample mixture to a method of assaying acyl-L-carnitines including acetyl-L-carnitine and propionyl-L-carnitine as defined above.

The present invention also provides Alcaligenes sp. No. 981 (FERM BP-2570) or a mutant thereof which is capable of producing an acylcarnitine esterase as defined above.

The present invention additionally provides a culture of Alcaligenes sp. No. 981 (FERM BP-2570) or a mutant thereof which is capable of producing an acylcarnitine esterase as defined above, in a culture medium which comprises a source of assimilable carbon, a source of assimilable nitrogen and mineral salts and which is free of other microorganisms.

Fig. 1 is a graph showing the optimum pH of an acylcarnitine esterase of the present invention.

Fig. 2 is a graph showing the stable pH range of an acylcarnitine esterase according to the present invention.

Fig. 3 is a graph showing the optimum temperature of an acylcarnitine esterase according to the present invention.

Fig. 4 is a graph showing the heat stability of an acylcarnitine esterase according to the present invention.

Fig. 5 is a graph comparing the hydrolyzing activity of an acylcarnitine esterase according to the present invention (indicated with a circle mark) with that of potassium hydroxide (indicated with a triangular mark) for octanoyl-L-carnitine.

Fig. 6 is a graph comparing the heat stability of an acylcarnitine esterase according to the present invention (indicated with a hollow circle mark) with that of an acylcarnitine esterase obtained from a rat liver (indicated with a solid circle mark).

Fig. 7 is a graph comparing the hydrolyzing activity of an acylcarnitine esterase according to the pressent invention (indicated with a hollow circle mark) with that of an acylcarnitine esterase obtained from a rat liver (indicated with a solid circle mark) for octanoyl-L-carnitine.

Fig. 8 is a graph showing the applicability of an acylcarnitine esterase according to the present invention to quantitative determination of acetyl-L-carnitine.

There is no special restriction for the acylcarnitine esterase-producing bacterium, so long as it belongs to the genus Alcaligenes and is capable of producing the above acylcarnitine esterase. One special example thereof is the bacterium of bacterial strain No. 981, which has been isolated by the inventors and has been shown as capable of being employed most effectively for realizing the present invention (hereinafter, this bacterial strain is called sometimes referred to as the present bacterial strain). The bacteriological properties of this bacterium are:

### a) Morphological properties

Observing the isolated bacterium after cultivation at 28 - 30 °C for 18 - 24 hours gave the following results:

It is a straight or somewhat curved rod-like bacterium having round ends existing in an isolated or double linked state or, seldomly, in a short chain. No spore formation, moving with peripheral flagella, no polymorphism, size of Zn 0.4 - 0.6 × 1.2 - 2.5 µm.

### b) Growth on various media

Observation by cultivating at 28 - 30 °C for 18 - 24 hours in various culture media gave the following results:
① Nutrient agar slant medium:
   The growth is better, growing in filiform. Wet and lustrous in appearence and ochre colored with no production of soluble pigment.
② Nutrient agar plate medium:
   A circular, convex and fully rimmed colony with a smooth wet surface; ochre to light ochre colored. No production of soluble pigment.
③ Liquid medium (aqueous peptone)
   Growth is better, turbid uniformly. In a long term culture (over 40 hours), formation of pellicle is observed.
④ BCP milk medium
   Becomes alkaline after 4 - 5 days.

### c) Physiological properties

In the following, the marks denote:
+ : positive,
(+): weakly positive,
- : negative,

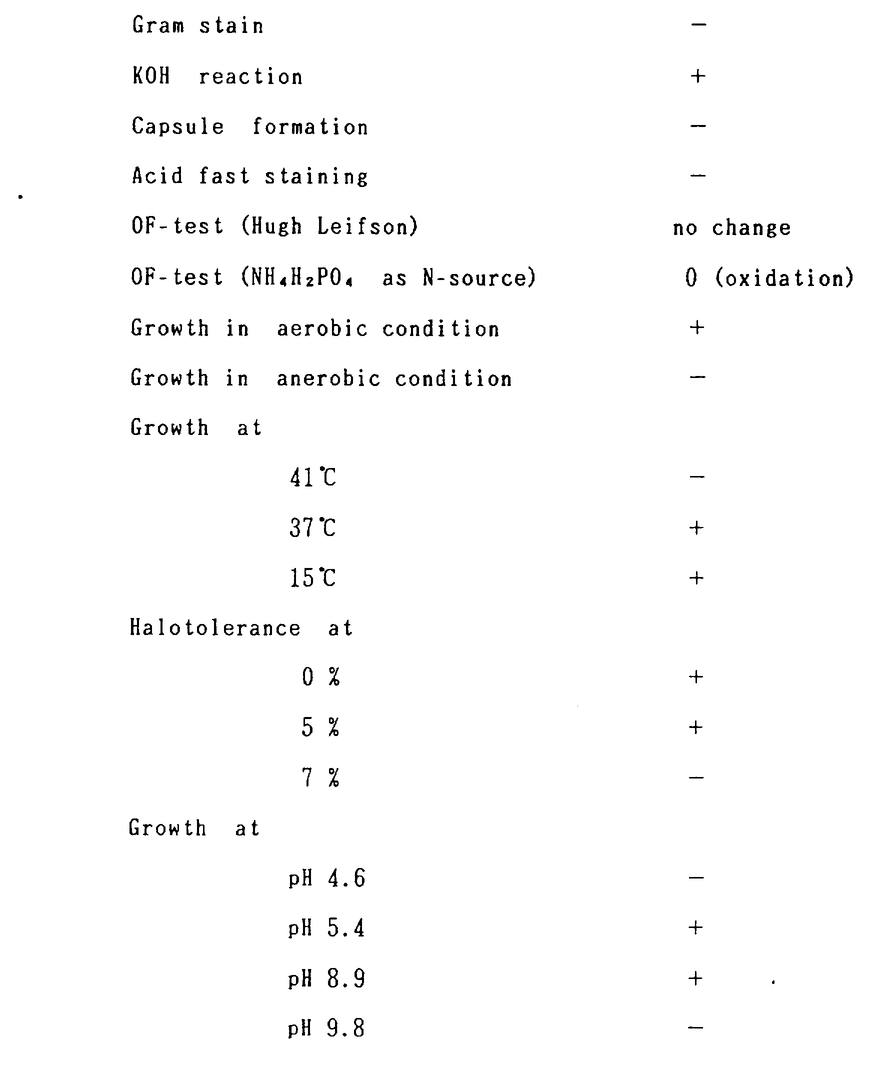

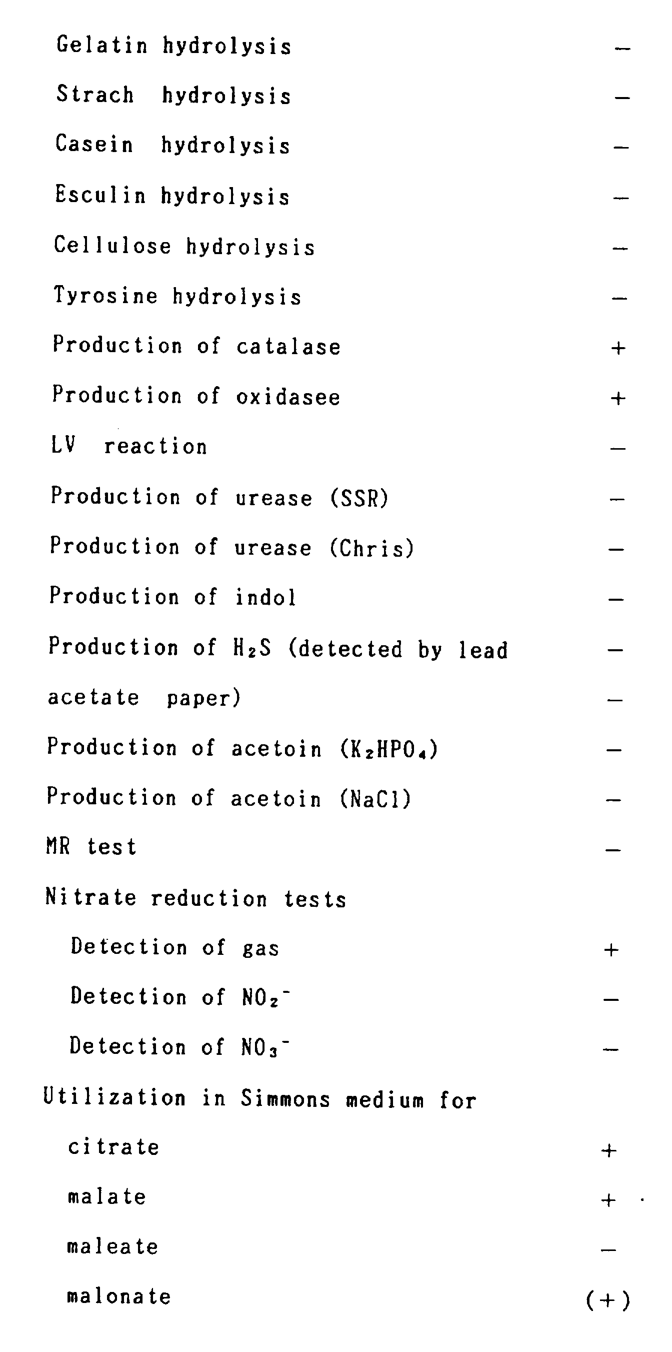

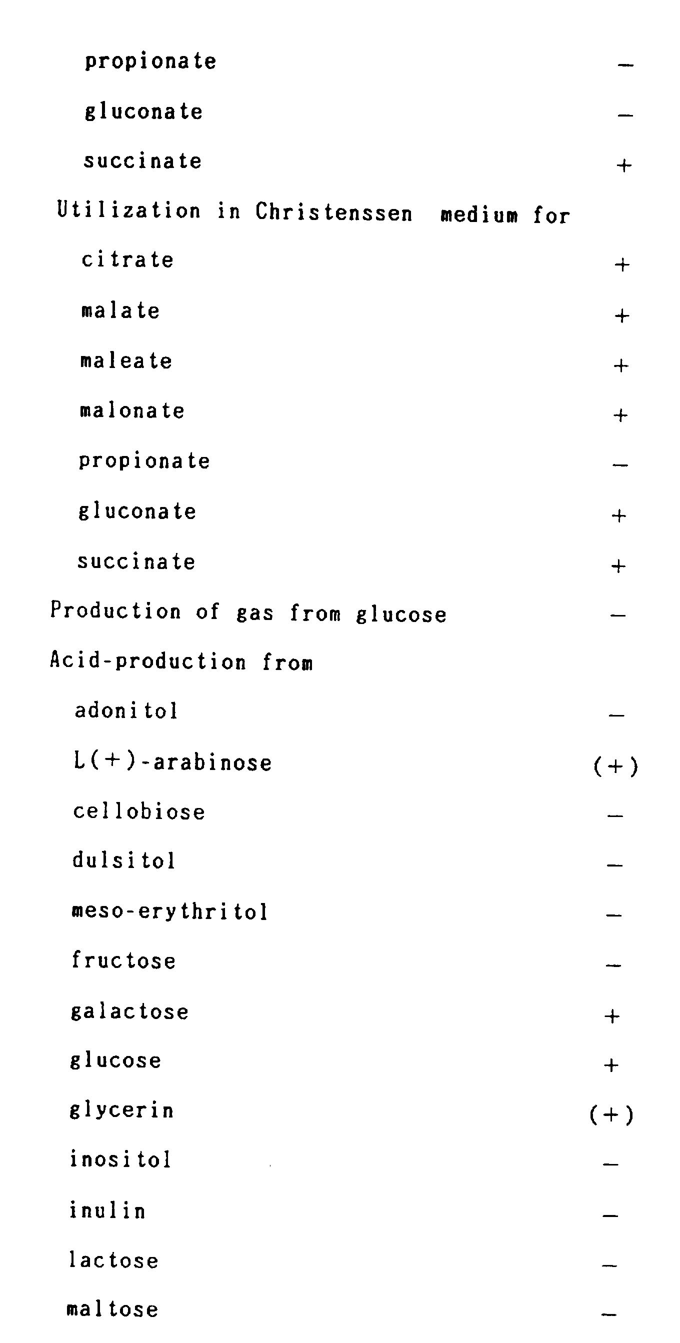

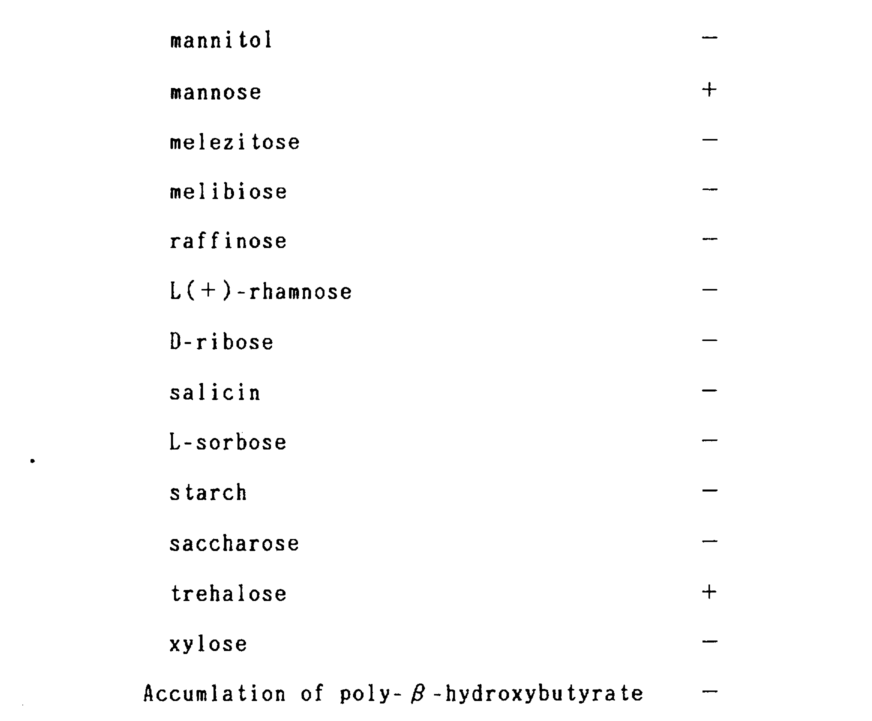

### d) Utilization of carbon sources

Examining the utilization of various carbon sources by cultivating in a liquid culture medium (pH 7.0) containing 5 g of a carbon source, 5 g of NaCl, 0.2 g of MgSO₄ · 7H₂O, 1.0 g of NH₄H₂PO₄ and 1 liter of distilled water gave the following results:

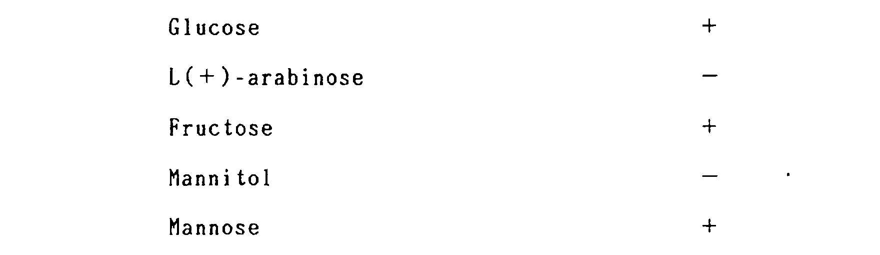

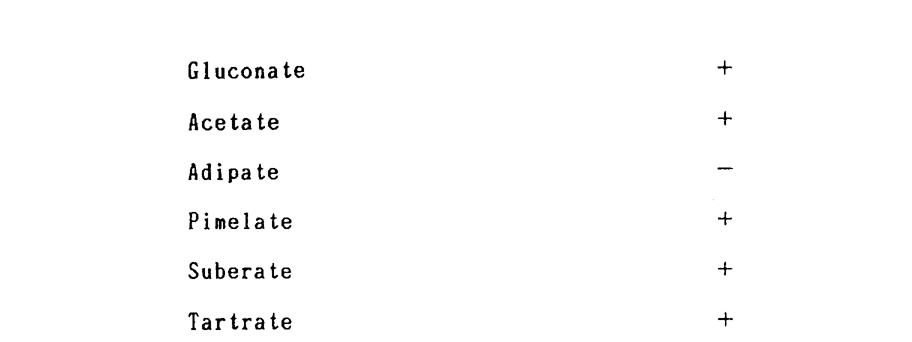

For the identification of the bacterium described above, the procedures taught in "Guide for the Identification of Bacteria in Medicine": "Microbiological method ", Vol 3, 2nd Ed. were employed. Identification was realized by comparison of the experimental results with the data given in, for example, "Bergey's Manual of Determinative Bcteriology", 8th Ed., "Bergey's Manula of Systematic Bacteriology " , Vol. 1 (1984) and ibid., Vol. 2 (1986).

The principal characteristic properties of the present bacterial strain may be expressed as a Gram negative rod-shaped bacterium moving with its peripheral flagella, producing catalase and oxidase and not producing acid from glucose in a medium containing peptone (Hugh-Leifson) but causing oxidative decomposition of glucose with production of acid, showing no formation of spores and no polymorphism and growing under aerobic condition.

Gram negative bacetria which are characterized by rod-shaped bacterial cells and growing under aerobic condition and moving with peripheral flagella are those in the genera Alcaligenes, Chromobacterium and Flavobacterium. Bacteria of the genus Chromobacterium produce purple colored pigment and those of the genus Flavobacterium produce yellow colored pigment. Since the bacterial strain according to the prsent invention does not produce any pigment, it is evident that it belongs to the genus Alcaligenes.

In order to discriminate which species of Alcaligenes the present bacterial strain belongs to, the characteristic properties of the present bacterial strain were compared with those of three bacterial species given in "Bergey's Manual of Systematic Bacteriology", Vol. 1, (1984), namely, Alcaligenes faecalis (hereinafter this is sometimes represented by "F "), Alcaligenes denitrificans subsp. denitrificans (hereinafter this is sometimes represented by "D ") and Alcaligenes denitrificans subsp. xylosoxidans (hereinafter this is sometimes represented by "X "). The results are given below.

In the following comparison, denotation by the mark " + " means a positive probability of over 90 %, the mark " - " means a negative probability of over 90 % and the mark "d " means that the property is judged to be neither positive nor negative.

| Properties | F | D | X | This strain |
|---|---|---|---|---|
| Production of oxidase | + | + | + | + |
| Reduction of nitrate | - | + | + | + |
| Reduction of nitrate | + | + | + | + |
| Gelatin hydrolysis | - | - | - | - |
| Production of acid in OF-medium for | | | | |
| Xylose | - | - | + | - |
| Glucose | - | - | + | - |
| Production of acid in culture media with no peptone for | | | | |
| Xylose | | | + | - |
| Glucose | | | + | + |
| Utilization of carbon source for | | | | |
| Glucose | - | - | + | + |
| L(+)-arabinose | - | - | - | - |
| Fructose | - | - | d | + |
| Mannitol | - | - | - | - |
| Mannose | - | - | d | + |
| Gluconate | - | + | + | + |
| Acetate | + | + | + | + |

From the above comparison, it was observed that the present bacterial strain has properties which are in concordance with those of Alcaligenes subsp. xylosoxidans for many items but are different therefrom for acid productivity in OF culture media and for no production of acid from xylose.

Accordingly, the present bacterial strain was judged to be distinct from these known bacterial species and, therefore, was deposited in Institute of Microbiological Engineering of the Agency of Industrial Science and Technology, Japan, under the denotation of Alcaligenes sp. No. 981 with the Deposition No. Bikoken Jo-ki 2570 (FERM BP-2570).

To the acylcarnitine esterase of the present invention, an acylcarnitine esterase-producing bacterium belonging to the genus Alcaligenes is first cultivated in an adequate culture medium.

An example of the acylcarnitine esterase-producing bacterium is Alcaligenes sp. No. 981. Since, however, the bacteriological properties of a bacterium can in general vary, every bacterial strain belonging to the genus Alcaligenes and capable of producing an acylcarnitine esterase of the present invention may be employed. Such bacterial strains may be those obtained from artificial mutation induced by UV irradiation, radiation, by the use of a mutagen agent such as, N-methyl-N-nitro-N-nitrosoguanidine or ethyl methanesulfonate, or may be those obtained from natural mutation.

The culture of the bacterium can be carried out under the conditions employed in general for bacterial culture. It is preferable, however, to carry out the culture in a culture medium containing an acyl-L-carnitine. As the acyl-L-carnitine, for example, octanoyl-L-carnitine, which is inexpensive, is preferably used (0.1 - 1 %, based on the weight of the culture medium). A nutrient culture medium comprising a carbon source capable of being anabolized by the bacteria, a nitrogen source capable of being digested by the bacteria and, on requirement, inorganic salts may be employed as the culture medium.

Examples of the carbon source are glucose, fructose, saccharose, sucrose, molasses and olive oil, either alone or in combination.

Examples of the nitrogen source are peptone, meat extract, yeast extract, corn steep liqour and choline hydrochloride, either alone or in combination.

In addition, inorganic salts, such as phosphate and salts of magnesium, calcium, potassium, sodium and heavy metals, such as iron and manganese, may be employed on requirement.

It is of course possible to employ other carbon sources and nitrogen sources capable of being anabolized or digested by the bacteria.

The culture is effected under an aerobic condition, preferably with shaking or aeration agitation. It is preferable for industrial practice to employ a submerged aeration culture with agitaion.

While the temperature for the culture may be varied within the range in which the acylcarnitine esterase-producing bacterium can grow under production of the esterase, it is usually preferable to employ a temperature of from 18 to 37°C, especially around 28°C. While the duration of the culture may be different for each specific culture condition, the culture may be terminated at an adequate stage at which the maximum production of the esterase is reached, usually after 1 to 3 days.

The culture conditions, such as composition and liquid nature of the culture medium, temperature, agitation rate and aeration rate, may be adjusted and selected so as to attain preferred results in accordance with each specific bacterial strain employed and external conditions. If foaming occurs in a liquid culture, an antifoaming agent, such as a silicone oil or vegetable oil, may be employed.

The acylcarnitine esterase produced is retained mainly within the bacterial cells. Therefore, the bacteria cells are collected from the culture product mixture by an appropriate means, such as filtration or centrifugation, and the collected bacteria cells are then subjected to a cell breaking treatment, such as mechanical breaking by ultrasonication, treatment with a French press, glass beads treatment or freezing-thawing, or an enzymatic digestion using, for example, lysozyme, or an appropriate combination of these mechanical and enzymatic means, to obtain a crude liqour containing the acylcarnitine esterase.

From the crude extract purified acylcarnitine esterase can be obtained by processes known for isolating and purifying proteins and enzymes. Thus, the recovery of the esterase can be attained by, for example, the so-called salting-out method by adding, for example, ammonium sulfate, sodium sulfate, sodium sulfate, potasium phosphate or aluminum chloride, to the crude extract containing the acylcarnitine esterase. The precipitate thus obtained may further be purified, on requirement, using various molecular sieves or by chromatography, electrophoresis, ultracentrifugation or a combination of these. In practice, the purification can be realized by making use of the properties of the acylcarnitine esterase to be isolated. For example, the precipitate obtained as above is first dissolved in water or in a buffer solution and, after dialyzing it as required, the solution is subjected to a molecular sieve chromatography using an ion-exchange resin, such as DEAE-cellulose, DEAE-Sephacel, DEAE-Sepharose or DEAE-Sephadex A-50 (products of the Pharmacia Corp.) or DEAE-Toyopearl (Toso Corp.), or using a gel filtration medium, such as Sephadex G-100 and G-75 or Sephacryl S-200. Combinations of these purification means may also be employed. The purified solution thus obtained can be stabilized by adding a stabilizing agent, for example a sugar, such as mannitol, saccharose or sorbitol; an aminoacid, such as glutamic acid a glycine; or bovine serum albumin as a peptide or protein, whereupon the solution is subjected to, for example, lyophilization, to obtain purified acylcarnitine esterase.

The characteristic properties of the isolated acylcarnitine esterase according to the present invention are :

### 1) Enzymatic activity

It catalyzes the hydrolysis reaction of an acyl-L-carnitine with water to form L-carnitine and the corresponding liberated free fatty acid: $\text{Acyl-L-carnitine + H₂O → Fatty acid + L-carnitine}$

### 2) Molecular weight

$\text{63,00 ± 7,000}$

The molecular weight was determined by chromatography using TSK Gel G3000 SW (a product of Toso Corp.) on a 0.75 × 60 cm column using an eluent consisting of a 0.1 M phosphate buffer solution (pH 7.0) containing 0.2 m NaCl using the following standard molecular weight markers supplied from Oriental Yeast Co. Ltd:

| Molecular Weight | Compound |
|---|---|
| 12,400 | Cytochrome C |
| 32,000 | Adenylate kinase |
| 67,000 | Enolase |
| 142,000 | Lactate dehydrogenase |
| 290,000 | Glutamate dehydrogenase |

### 3) Isoelectric point

$\text{pH 5.1 ± 0.5}$

The isoelectric point was determined by the so-called isoelectric focussing method using a carrier ampholite under a constant voltage of 700 V for 40 hours, whereupon the solution was subjected to a fractionation and each fraction was examined for enzymatic activity.

### 4) Km value

Solutions of acylcarnitines in 100 mM tris-HCl buffer solution (pH 8.0) were prepared in such a manner that the concentrations of each acylcarnitine was 1 × 10⁻⁵ M, 2 × 10⁻⁵ M, 3 × 10⁻⁵ M, 5 × 10⁻⁵ M, 10 × 10⁻⁵ M, 20 × 10⁻⁵ M and 40 × 10⁻⁵ . The Km value for each specific acycarnitine was determined. Here, for the acylcarnitines, either those obtainable commercially or those prepared from L-carnitine (a product of Sigma) according to the procedures of Bohemer & Bremer described in "Biochim. Biophys. Acta", 152, 559 - 567 (1968) were employed.

| Acylcarnitine | Km Value |
|---|---|
| Acetylcarnitine | ca. 4 × 10⁻⁵ M |
| Propionylcarnitine | ca. 3 × 10⁻⁵ M |
| Hexanoylcarnitine | ca. 2 × 10⁻⁵ M |
| Octanoylcarnitine | ca. 2 × 10⁻⁵ M |
| Decanoylcarnitine | ca. 2 × 10⁻⁵ M |
| Lauroylcarnitine | ca. 2 × 10⁻⁵ M |
| Myristoylcarnitine | ca. 2 × 10⁻⁵ M |
| Palmitoylcarnitine | ca. 2 × 10⁻⁵ M |
| Stearoylcarnitine | ca. 2 × 10⁻⁵ M |

### 5 ) Substrate specificity

Acylcarnitine solutions in 100 mM tris-HCl buffer solution (pH 8.0) were prepared so that the concentration of each acylcarnitine was adjusted to 0.5 mM. Each of the acylcarnitine solutions was subjected to the enzymatic hydrolysis reaction at 37 °C for 10 minutes, whereupon the amount of L-carnitine formed was determined by the L-carnitine analysis described below in order to compare the enzymatic activity for the carnitines. It was found that the highest activity was attained for octanoyl-L-carnitine. Moreover, it was also found that the acylcarnitine esterase which originated from the liver of a rat did not exhibit enzymatic activity for acetyl-L-carnitine or propionyl-L-carnitine, whereas the esterase according to the present invention had a sufficient activity for these short chain acylcarnitines.

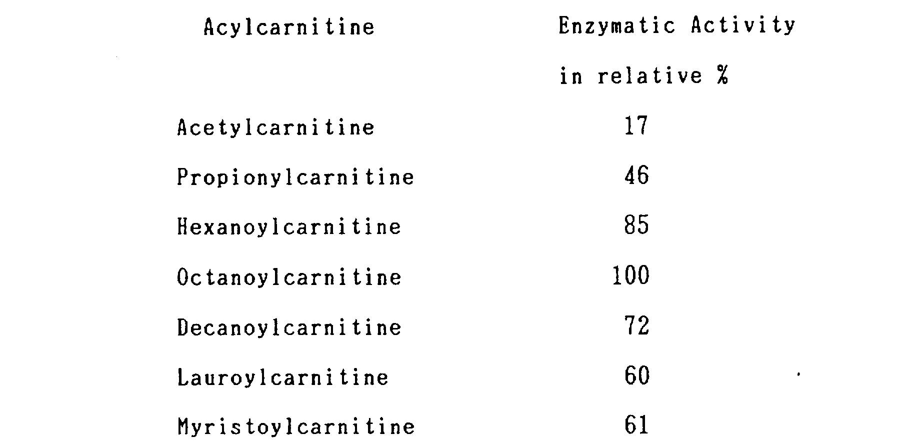

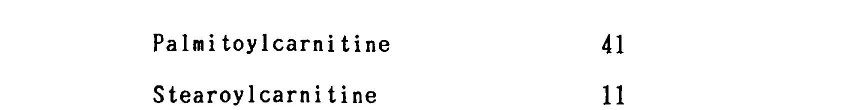

### 6) Optimum pH

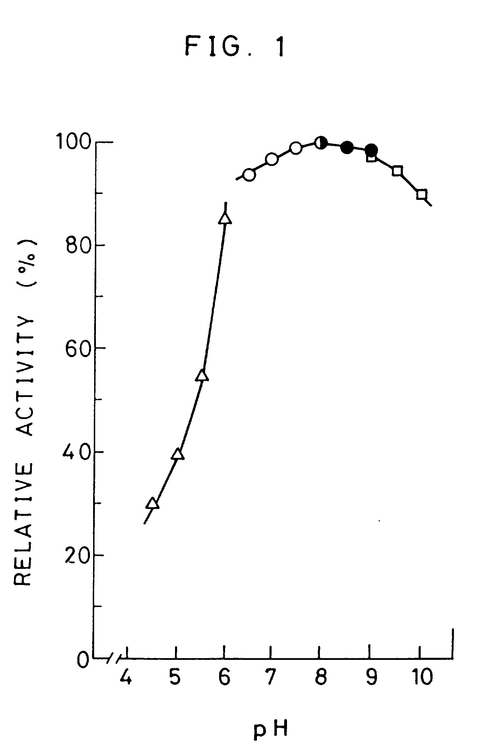

Octanoyl-L-carnitine solutions in 100 mM acetate buffer solution (pH 4.5 - 6.0), in 100 mM phosphate buffer solution (pH 6.5 - 8.0), in 100 mM tris-HCl buffer solution (pH 8.0 - 9.0) or in 100 mM glycine /NaOH buffer solution (pH 9.0 - 10.0) were prepared so as to adjust the concentration of octanoyl-L-carnitine to 0.5 mM for each of the solutions. Each of these solutions was subjected to an enzymatic hydrolysis reaction at 37°C for 10 hours and the amount of L-carnitine formed was determined. The results of the analysis of L-carnitine are given in the graph of Fig. 1, which shows that the optimum pH is about 8.0. High enzymatic activities above 90 % were reached within a wide pH range of from 6.5 to 9.5.

In the graph of Fig. 1, the marks Δ, O, ● and □ correspond to acetate buffer solution, phosphate buffer solution, tris-HCl buffer solution and glycine /NaOH buffer solution respectively.

### 7) pH Stability

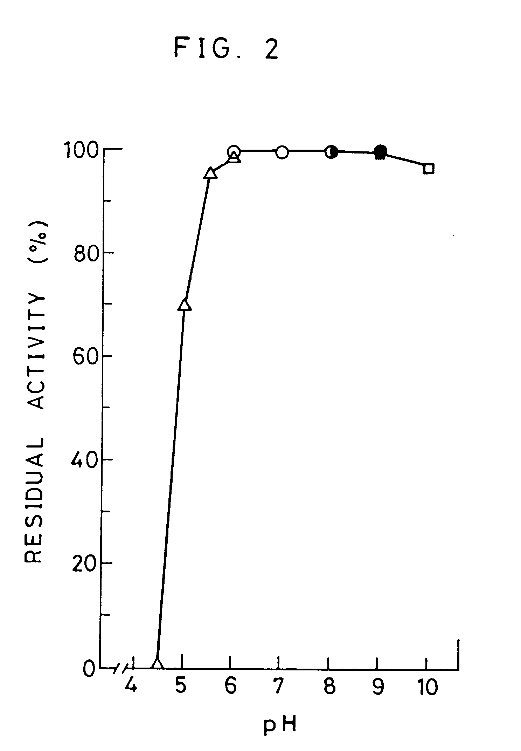

Solutions of the esterase according to the present invention in 100 mM acetate buffer solution (pH 4.5 - 6.0), in 100 mM phospahte buffer solution (pH 6.5 - 8.0), in 100 mM tris-HCl buffer solution (pH 8.0 - 9.0) or in 100 mM glycine/NaOH buffer solution (pH 9.0 - 10.0) were prepared so that a concentration of the esterase of 0.1 Unit/ml was obtained for each of the solutions. Each solution was heat treated at 60°C for 30 minutes, whereupon the residual activity was determined. The results are given in Fig. 2, which shows that the esterase was stable in the phosphate buffer solution of pH 7.5 and in the tris-HCl buffer solution of pH 8.5. High enzymatic activities above 90 % were reached within a wide pH range of from 5.5 of the acetate buffer solution to 10.5 of the glycine/NaOH buffer solution.

In the graph of Fig. 2, the marks Δ, O, ● and □ correspond to acetate buffer solution, phosphate buffer solution, tris-HCl buffer solution and glycine /NaOH buffer solution respectively.

### 8) Optimum temperature

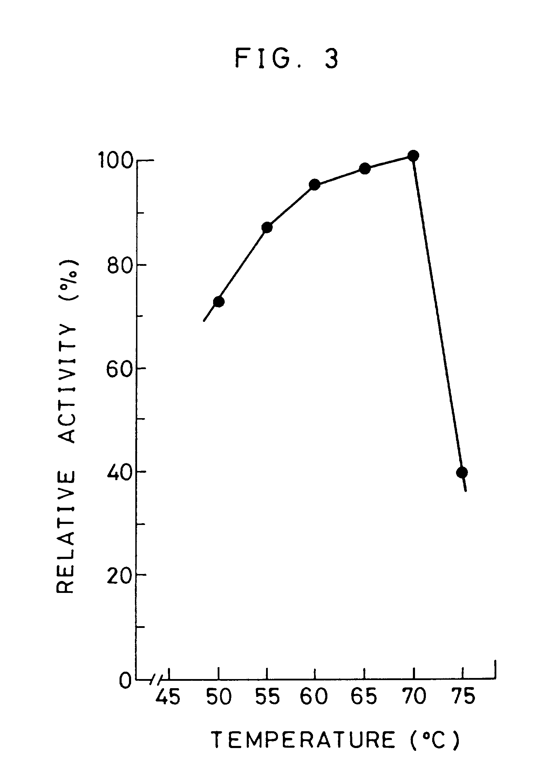

Using a 0.5 mM octanoyl-L-carnitine solution of pH 8.0 in 100 mM tris-HCl buffer solution, the enzymatic hydrolysis reaction was carried out at 50, 55, 60, 65, 70 or 75°C each for 10 minutes, whereupon the amount of L-carnitine formed was determined. The results of analysis are given in the graph of Fig. 3, which shows that the maximum activity is at 70°C.

### 9) Heat stability

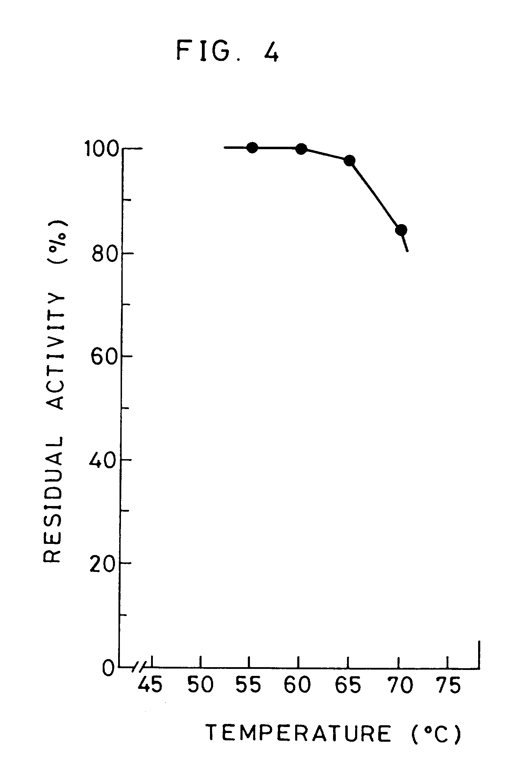

A set of solutions of the esterase according to the present invention were prepared using 100 mM tris-HCl buffer solution (pH 8.0) so that each of the solutions had a concentration of the esterase of 0.10 Unit/ml. Each of these solutions was heat treated either at 55, 60, 65 or 70°C for 30 minutes, before it was examined for its residual enzymatic activity. The results are given in the graph of Fig. 4, which shows that the esterase is stable up to 60 °C.

### 10) Assay method of acylcarnitine esterase

### ①Reaction Mixture

| | |
|---|---|
| tris-HCl buffer solution (pH 8.0): | 100 mM |
| Octanoyl-L-carnitine: | 0.5 mM |

### ②Procedures

1 ml of the above reaction solution is placed in a small test tube and incubated at 37°C for 5 minutes. 0.05 ml of esterase solution under adequate dilution is added thereto and the resulting solution is incubated at 37°C for 15 minutes, whereupon the reaction is terminated at once by transfering the test tube into a boiling water bath and subjecting the solution to incubation for 15 seconds to prepare a sample solution to be inspected. The amount of L-carnitine produced is determined according to the L-carnitine determination method described below to assess the activity of the acylcarnitine esterase.

### ③ Calculation equation

$\text{Unit/ml=} \frac{{\text{A}}_{\text{1}} {\text{-A}}_{\text{0}}}{\text{21.7}} \text{×} \frac{\text{1}}{\text{15}} \text{×} \frac{\text{3.05}}{\text{0.05}} \text{×} \frac{\text{1.05}}{\text{0.05}} \text{×Z}$ (in which Z is the dilution factor and the numerals 21.7 and 15 correspond to the molecular extinction coefficient in cm / µmol and the reaction time in minutes respectively)

### ④ Method for analyzing L-carnitine (hereinafter designated as analysis method A)

### (a) Composition of the reaction solution

| | |
|---|---|
| Tris-HCl buffer solution (pH 9.0) | 100 mM |
| NAD ⁺ | 1 mM |
| Diaphorase (of Toyo Jozo Co. Ltd.) | 5 Units |
| L-carnitine dehydrogenase (obtained from Alcaligenes sp. No 981, supplied from Toyo Jozo) | 15 Units |
| KCl | 100 mM |
| NBT (of Wako Pure Chemical Ind.) | 0.025 % |
| Polyoxyethylene (20) sorbitan-monooleate (of Wako Pure Chemical Ind.) | 0.5 % |

### (b) Procedures

1 ml of the above reaction solution is placed in a small test tube and incubated at 37°C for 5 minutes . 0.05 ml of the esterase solution to be examined is then added and the resulting solution is incubated for 2 minutes, whereupon 2 ml of 0.1 N aq. HCl are added thereto and the solution is examined at A_{550 nm} to obtain the absorbance A₁. The absorbance A₀ for a blank test solution without addition of the esterase solution is determined in the same way.

### (c) Calculation equation

$\text{n mole/ml=} \frac{{\text{A}}_{\text{1}} {\text{-A}}_{\text{0}}}{\text{21.7}} \text{×} \frac{\text{3.05}}{\text{0.05}}$

As described above, the acylcarnitine esterase according to the present invention acts as an enzyme not only for acyl-L-carnitines of medium to long chain lengths, but also for short chain acyl-L-carnitines for which the conventional acylcarnitine esterases are ineffective. Therefore, acyl-L-carnitines contained in a sample solution of the substances to be examined, such as human serum, can be determined by subjecting the sample solution to an enzymatic hydrolysis with the acylcarnitine esterase according to the present invention which exhibits a substrate-specificity not only to acyl-L-carnitines of medium to long chain acylcarnitine but also to short chain acylcarnitines (acetyl-L-carnitine and propionyl-L-carnitine) and, then, determining the amount of the fatty acids or L-carnitine thus formed, by an analysis technique known per se.

If there is free L-carnitine in the sample solution in addition to the acyl-L-carnitines, the analysis of the acyl-carnitines may be realized by, for example, determined preliminarily the amount of free L-carnitine by the analysis technique described below; decomposing the free L-carnitine with a reducing coenzyme, such as by a system in which NADH is converted into the oxidized form, and heating the reaction mixture, whereupon the acyl-L-carnitines are analysed; or converting all the acyl-L-carnitines into free L-carnitine using the esterase according to the present invention and determining the total L-carnitine including the L-carnitine originally present by the known analysis technique.

There is no special restriction for the conditions of the reaction of the acyl-L-carnitines in the sample solution with the acylcarnitine esterase according to the present invention, so long as sufficient hydrolysis of the acyl-L-carnitines in the sample solution is attained. It is recommended, however, to effect the reaction by incubating the esterase-inoculated sample mixture at a temperature of, for example, 37 °C for a time of, for example, 5 - 30 minutes. The enzymatic hydrolysis reaction may preferably be terminated by heating the reaction solution to a temperature above 80°C .

By the enzymatic hydrolysis effected as above, the corresponding fatty acid and L-carnitine are liberated from the acyl-L-carnitine. Therefore, the amount of the acyl-L-carnitine can be detetcted by quantitatively analyzing either the amount of the free fatty acid or that of the L-carnitine.

The quantitative determination of the fatty acid can be effected by an analysis technique known per se. Ordinary analysis techniques include, for example, liquid chromatography and gas chromatography. The known techniques for quantitatively determining L-carnitine include, for example, a colorimetric method using a carnitine actyltransferase (CAT), acetyl CoA and 5, 5'-dithio-bis-nitrobezoic acid (DTNB) reported in the literature "J. Biol. Chem. ", 238, 2509 (1963) and "J. Lipid Research" 5, 184 - 187 (1964); a radioisotope method using ¹⁴C - or ³H-labelled acetyl-CoA and CAT reported in the literature " Clin. Chim. Acta " , 37, 235 - 243 (1972) and "J. Lipid Research " , 17, 277 - 281 (1976); a carnitine dehydrogenase method using a carnitine dehydrogenase NAD⁺ as reported in " European J. Biochem. ", 6, 196 - 201 (1968) and a fluorimetric method using acetyl-CoA, CAT and N- [p- (2-benzimidasol-yl)-phenyl]-maleimide (BIPM) as reported in the 61st year's Research Report of the Institute of Neuropathy of the Ministry of Health and Welfare, Japan, 315 - 318 (1986). Among them, the carnitine dehydrogenase method is recommended. This method comprises subjecting the sample solution to the action of a reagent containing an L-carnitine dehydrogenase and one of nicotinamide adenine dinucleotide group (NAD group) or thio nicotinamide adenine dinucleotide group (thio-NAD group) with, if necessary, a non-ionic detergent and determining the amount of the reduced NAD group compound or the reduced thio-NAD group compound thereby formed. Examples of compound is in the NAD group are nicotinamide adenine dinucleotide (NAD), acetylpyridine adenine dinucleotide (acetyl-NAD) and nicotinamide hypoxantine dinucleotide (deamino-NAD). Examples of the compounds in the thio-NAD group are thionicontinamide adenine dinucleotide and thionicotinamide hypoxanthine dinucleotide .

While the analysis of the reduced NAD group compound or the reduced thio-NAD group compound formed by the reaction can be effected by a direct determination of the absorbance of the reacted sample solution, it is preferable to determine it by converting the reaction system into a formazan forming system represented by the following reaction schemes:
(1) L-carnitine dehydrogenase (LCD) reaction system 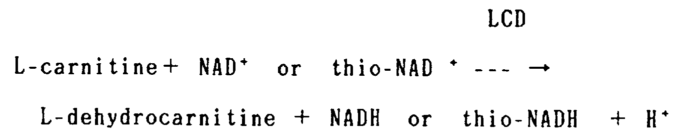
(2) Conversion reaction system $\text{Tetrazolium salt + NADH or thio-NADH + H⁺ ------------ → Formazan + NAD⁺ or thio-NAD ⁺}$diaphorase or
   phenazine derivative

The L-carnitine dehydrogenase to be employed according to the present invention is a known enzyme. It is recommended to use a bacteria-originated dehydrogenase produced by an L-carnitine dehydrogenase-producing bacterium. Examples of such L-carnitine dehydrogenase-producing bacteria are those of the genus Pseudomonas, such as Pseudomonas aeruginosa IFO 13130, Pseudomonas putida B-0781 (FERM P-5664) and Pseudomonas putida IF 03738, and those of the genus Xanthomonas, such as Xanthomonas translucens IFO 13558. The bacteria-originated L-carnitine dehydrogenase can be obtained according to the procedures described in the literature, such as, "European J. Biochem. ", 6, 196 - 201 (1968), ibid. 10, 56 - 60 (1969), "Agric. Biol. Chem. ", 52 (1), 249 - 250 (1988), by cultivating the bacteria, collecting the dehydrogenase from the culture product and purifying it. It can also obtained by means of a gene recombination technique by producing a recombinant host-cell having the L-carnitine dehydrogenase-producing gene of a bacterium mentioned above, cultivating it and collecting the dehydrogenase produced [see, for example, "Agric. Biol. Chem. " , 52 (3), 851 - 852 (1988)]. By way of observation, the bacterium Alcaligene sp. No. 981 also produces an L-carnitine dehydrogenase. It is preferable to carry out the culture in a culture medium containing carnitine.

In the analysis methods proposed above, an electron transfering agent, namely an agent capable of converting a tetrazolium salt, NAD-group (or thio-NAD group) and H ⁺ into formazan and NAD ⁺ s (or thio-NAD ⁺ s), such as diaphorase or a phenazine derivative is ussed.

Diaphorase is a known enzyme and is commercially available.

As the phenazine derivatives, there may be enumerated phenazine methosulfate, meldola's blue and methoxyphenazine methosulphate.

As the tetrazolium salt to be employed in these analysis methods, there may be enumerated 3, 3'-(3, 3'-dimethoxy-4, 4′-biphneylene)-bis-[2- (p-nitrophenyl)-5-phenyl-tetrazolium chloride], named commonly as nitrotetrazolium (NBT); 3, 3'-(3, 3'-dimethoxy-4, 4'-biphenylene)-bis [2, 5-bis(p-nitrophenyl)-tetrazolium chloride]; 3, 3'-(3, 3′-dimethoxy-4, 4′-biphenylene)-bis[2, 5-diphenyl-tetrazolium chloride] ; 2-(p-nitrophenyl)-3-(piodophenyl)-5-phenyl-tetrazolium chloride (INT); 3-(4, 5-dimethyl-2-thiazolyl)-2, 5-diphenyl-tetrazolium chloride (4, 5-MTT); 3-(4, 5-dimethyl-2-triazolyl)-2, 4-diphenyl-tetrazolium chloride (MTT); 2, 2'-5, 5'-tetra-(p-nitrophenyl)-3, 3'- (3-dimethoxy-4-diphenylene)-ditetrazolium chloride (TNBT) ; 2, 3, 5-triphe-nyl-tetrazolium chloride (TT); and neotetrazolium chloride (NT).

Water soluble non-ionic detergents which have HLB values above 10 and exhibit no negative influence disturbing the reaction are employed as the non-ionic detergent to be added to the reaction system. It is preferable to employ non-ionic detergents having HLB values of from about 11 to 17. Examples of such non-inonic detergents include polyoxyethylene alkyl ethers, such as polyoxyethylene oleyl ether, polyethylene cetyl ether, polyoxyethylene stearyl ether lauryl, polyoxyethylene lauryl ether, polyoxyethylene hexadecyl ether and polyxyethylene tridecyl ether; polyoxyethylene alkyl aryl ethers, such as polyoxyethylene nonylphenyl ether and polyoxyethylene octyl phenyl ether; polyoxyethylene polyoxypropylene ethers, such as polyoxyethylene polyoxypropylene cetyl ethers ; polyoxyethylene alkyl esters, such as polyoxyethylene monostearate, polyoxyethylene monolaurate and polyoxyethylene monooleate; sorbitan derivatives, such as sorbitan monolaurate, sorbitan monopalmitate, sorbitan monostearate, sorbitan tristearate, sorbitan monolaurate, sorbitan sesquioleate and sorbitan trioleate; glycerin propylene glycol fatty acid esters, such as glycerin monostearate, polypylene glycol monostearate and glycerin monooleate; polyoxyethylene sorbitan fatty acid esters, such as polyoxyethylene sorbitan monolaurate, polyoxyethylene sorbitan monopalmitate, polyoxyethylene sorbitan monostearate, polyoxyethylene sorbitan tristearate, polyoxyethylene sorbitan monooleate and polyoxyethylene sorbitan trioleate ; polyoxyethylene sorbitol fatty acid esters, such as polyoxyethylene sorbitol monolaurate, polyoxyehtylene sorbitol tetraoleate and polyoxyethylene sorbitol hexastearate; polyoxyethyleneglycerin fatty acid esters, such as polyoxyethylene glycerin monostearate etc; polyoxyethylene alkylamines, such as polyoxyethylene stearylamine ; polyoxyethyleneamides, such as polyoxyethylene stearylamide ; fatty acid alkanolamides, such as lauric acid diemethanolamide and coconut oil fatty acid diethanolamide ; polyoxyethylene castor oil derivatives, such as polyoxyethylene-hydrogenated castor oil derivatives ; primary alcohol ethoxylates, such as Adekanol (a product of Asahi Denka Kogyo) ; secondary alcohol ethoxylates, such as Adekatol (a product of Asahi Denka Kogyo) and polyoxypropylene polyoxyethylene ethers of ethylenediamide, such as Tetronic (a product of Asahi Denka Kogyo). Among them, polyoxyethylene polyoxypropylene ethers, such as Pronic F-68 (a product of Asahi Denka Kogyo); polyoxyethylene sorbitan fatty acid esters, such as polyoxyethylene (20) sorbitan monooleate (a product of Wako Pure Chemical Ind.); sorbitan fatty acid esters; and secondary alcohol ethoxylates, such as Adekatol (Asahi Denka Kogyo) are preferred. These non-ionic detergents may be employed alone or in combination of two or more.

The concentrations of the enzyme and the reagents to be employed in carrying out the quantitative determination of L-carnitine in the sample solution may, in general, be selected from the ranges given below :

| Component | Concentration |
|---|---|
| L-carnitine dehydrogenase | 1 - 30 U/ml |
| NAD⁺ s (or thio- NAD⁺ s) | 0.1 - 5 mM |
| Diaphorase | 0.5 - 50 U/ml |
| Tetrazolium salt | 0.01 - 0.1 % |
| Non-ionic detergent | 0.1 - 5 % |

The enzymes and other requisite reagents may be stored as an aqueous solution in a separate system or in a combination system composed of two or more of the components or as dry powder. It is advantageous to employ freeze drying for storing them in the form of a dry powder. In order to assure stable storage of the enzymes and other requisite reagents, it is preferable to store each of them either alone or in combination with other components having no negative influence on the stability. For example, it is not preferable to store the L-dehydrogenase in combination with the non-inonic detergent, the NAD⁺ s or thio-NAD⁺ s in combination with the tetrazolium salt, the electron transfer agent in combination with the tetrazolium salt and the non-ionic detergent in combination with the tetrazolium salt in one single system for long period of time.

The reaction for the quantitative analysis of the acyl-L-carnitines mentioned above can usually be effected at a temperature of around 37 °C for at least 1 minute. If the reaction brings forth any turbidity in the reaction mixture, an appropriate additive such as KCl or NaCl may be incorporated in the reaction system to prevent such turbidity.

The amount of L-carnitine in the reaction system can be determined by colorimetry using a light of a wavelength around the specifc absorption band of the formazan formed by the reaction, namely at 500 - 550 nm.

It is also possible to carry out the analysis of L-carnitine with a reagent containing an L-carnitine dehydrogenase and a combination of either (1) one of the NAD group and one of the reduced thio-NAD group compounds or (2) one of the thio-NAD group and one of the reduced NAD group compounds, to effect the cycling reaction : 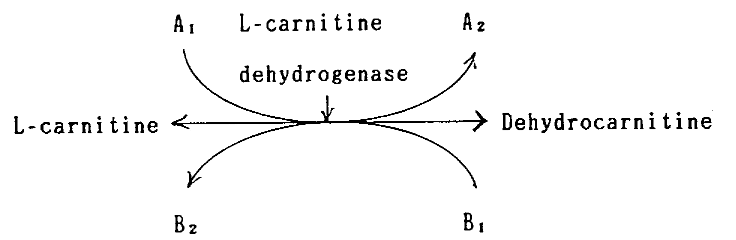 wherein A₁ is a NAD group or thio-NAD group compound, A₂ is the corresponding reduced form of A₁, B₁ is a reduced thio-NAD group compound when A₁ is a NAD group compound or is a reduced NAD group compound when A₁ is a thio-NAD group compound, and B₂ is the corresponding oxidized form of B₁; and, then, determining the amount of B₁ consumed during the cycling reaction or the amount of A₂ formed during the cycling reaction. This reaction system provides a very high sensitivity and is particularly preferable.

In this reaction system, it is necessary that at least either one of the combinations is a thio-coenzyme. For example, A₁ should be NAD when B₁ is thio-NADH, or A₁ should be thio-NAD when B₁ is NADH.

It is necessary that the amount of A₁ and B₁ is in excess to the amount of L-carnitine in the sample solution and this should also be in excess as compared with the Km value of the L-carnitine dehydrogenase for either of A₁ and B₁. It is preferable in particular that the amount of A₁ and of B₁ is about 20 - 10,000 times the amount of L-carnitine.

It is preferable in the analysis reagent for determining the amount of L-carniitne in the above-mentioned reaction system that the concentration of A₁ and B₁ are from 0.02 - 100 mM, especially 0.05 to 30 mM. The concentration of L-carnitine dehydrogenase is preferably 5 - 200 Units/ml, in particular, 10 - 150 U/ml. A higher concentration may also be permitted.

As concerns the L-carnitine dehydrogenase in preparing the analysis reagent for determining the amount of L-carnitine, those which have reactivity to the substrate, namely L-carnitine, may be employed in combination with a coenzyme, such as NADs (preferably NAD or thio-NAD). Such a reactivity can be confirmed using the combination of the coenzyme with the substrate. For example, it has benn confirmed that, when using L-carnitine as the substrate and thio-NAD as the coenzyme in 100 mM tris-HCl buffer solution (pH 9.0), the L-carnitine dehydrogenase produced from Alcaligenes sp. No 981 (supplied from Toyo Jozo) exhibits a relative enzymatic activity of about 15 % relative to the activity when using NAD as the coenzyme. The Km value for L-carnitine, NAD and thio-NAD were found to be 9.3 mM, 0.14 mM and 0.49 mM respectively, under the same conditions.

As concerns the reaction solution composition, it is recommended to select the combination of the two coenzymes suitably with consideration of, for example, the balance of the activities relative to each other for each specific L-carnitine dehydrogenase employed and to select the pH so as to cause the relative reaction rates of forward/reverse reactions to be as near to 1 as possible by considering the effect of the pH on the forward reaction and the reverse reaction rates.

In the analysis processes according to the present invention, an L-carnitine hydrogenase can be employed solely or in combination of two or more.

The quantitative determination of the amount of L-carnitine contained in the reacted sample solution using the analysis reagent for determining L-carnitine for the reaction system described above can be realized by, for example, adding to the reagent containing the above three essential components the reacted sample solution in an amount of 0.01 - 0.5 ml, carrying out the enzymatic reaction at a temperature of about 37°C and determining the amount of A₂ formed or the amount of B₁ consumed during the reaction at two points of time with a predetermined time interval. For example, a time interval of 1 minute, e.g., after 3 minutes and 4 minutes from the start of the reaction, or a time interval of 5 minutes, e.g., after 3 minutes and 8 minutes from the start of the reaction, may be employed, using spectrophotometry by observing the change in the absorbance during the time interval at the wavelength corresponding to the specific absorption band for A₂ or B₁ respectively. It is also possible to observe the change in the absorbance during the reaction by terminating the reaction after a predetermined time interval, such as after 10 minutes, from the start of the reaction. The amount of A₂ formed may be determined by measuring the increase in the absorbance at 400 nm [molar extinction cofficient = 11,200 M ^{- 1 cm - 1;} cf. " Methods in Enzymology ", Vol. 55, 261 (1979)], when A₂ is thio-NAD and B₁ is NADH, or by determining the amount of consumption of B₁ by detecting the decrease in the absorbance at 340 nm (molar extinction coefficient = 6220 M ^{- 1 cm - 1}) and comparing the detected value with the preliminarily obtained values for known concentrations of L-carnitine, in order to perform a real time assay of the amount of L-carnitine in the substance to be examined.

Since the above analysis method uses the enzymatic cycling reaction of L-carnitine itself in the sample solution, it scarcely suffers from any influence of co-existing substances in the sample solution. Thus it is permissible to dispense with the blank determination of the sample solution, resulting in convenient and simple to practice rate assay.

In this reaction system, it is also possible to determine A₂ or B₁ by other known methods for determining enzymes instead of the direct measurement of the absorbance.

The acylcarnitine esterase according to the present invenion can provide a way of determining the amount of short chain acyl-L-carnitine in the sample solution by a combined use thereof with the prior art acylcarnitine esterase, since the acylcarnitine esterase according to the present invention also has an enzymatic activity for short chain acyl-L-carnitines, namely acetyl-L- carnitine and propionyl-L-carnitine.

Thus, the present invention also provides a method of assaying acetyl-L-carnitine and propionyl-L-carnitine in a sample, which comprises:
(a) subjecting part of the sample to enzymatic hydrolysis with an acylcarnitine esterase which has no enzymatic activity for acetyl-L-carnitine and propionyl-L-carnitine but which exhibits substrate-specificity to acyl-L-carnitines of longer chain lengths and which catalyzes the hydrolysis reaction of one mole of acyl-L-carnitine of said longer chain lengths with one mole of water to form one mole of the corresponding fatty acid and one mole of L-carnitine (which esterase is denoted hereinafter as long chain acylcarnitine esterase) and then determining the amount of the fatty acids and/or L-carnitine thus formed, for example by an analysis technique known per se;
(b) subjecting another part of the sample to an assay of acyl-L-carnitines including acetyl-L-carnitine and propionyl-L-carnitine as defined above; and
(c) determining the difference in the analytical values obtained in steps (a) and (b).

While there is no special restriction as to the long chain acylcarnitine esterase to be employed in the process step (a) of the above-mentioned analysis method, it is recommended to employ an acylcarnitine esterase which originates from a rat.

The procedures in the above process steps (a) and (b) are essentially the same. The difference in the determined value between the process steps (a) and (b) corresponds to the amount of the short chain acyl-L-carnitines in the sample solution.

The present invention also provides a further method of assaying acetyl-L-carnitine and propionyl-L-carnitine in a sample, which comprises:
(a₁) subjecting the sample to enzymatic hydrolysis with a long chain acylcarnitine esterase and then subjecting the resultant reacted sample mixture to the action of an L-carnitine dehydrogenase with a coenzyme A, (wherein A₁ is a NAD or thio-NAD group compound) to convert A₁ into its reduced form A₂, and treating the resultant sample mixture to decompose A₂; and
(b₁) subjecting the resultant sample mixture to a method of assaying acyl-L-carnitines including acetyl-L-carnitine and propionyl-L-carnitine as defined above.

The enzymatic reaction in the process step (a₁) can be realized in the same manner as that of (a) of the foregoing analysis method. The decomposition of A₂ in the process step (a₁) of this analysis method is realized by warming or heating the reacted sample mixture under an acidic condition at a temperature of 37°C or higher, whereby all the L-carnitine, namely that derived from the longer chain acyl-L-carnitines plus that which existed originally, is removed from the reaction system by decomposition. Thus, by the decomposition of A₂, the sample mixture contains only the lower-L-carnitines, whereby the amount of the short chain acyl-L-carnitines can be determined by process step (b₁), which is essentially the same as that of (b) of the former analysis method.

The acylcarnitine esterase according to the present invention has a distict feature that it exhibits a substrate-specificity also to acetyl-L-carnitine and propionyl-L-carnitine, for which conventional acylcarnitine esterases have no substrate-specificity. In addition, the acylcarnitine esterase according to the present invention exhibits lower Km values for various acyl-L-carnitines and is superior also in stability. By making use of these superior features of the acylcarnitine esterase according to the present invention, advantageous and convenient assays of acylcarnitines can be proposed. The present invention proposes also an advantageous process for the production of such an acycarnitine esterase by making use of the excellent heat stability of the esterase, permitting easier attainment of purification with simple and economical large-scale production by aerobic cultivation.

The present invention is now further described in the following Examples :

### Example 1

### Culture of Alcaligenes sp. No. 981

100 ml of a liquid culture medium (pH 7.0) containing 0.2 % of KH₂HO₄, 0.4 % of K₂HPO₄, 0.05 % of M_{g}SO₄ · 7H₂O, 0.02 % of FeSO₄ · 7H₂O, 0.02 % of MnSO₄ · nH₂O and 0.1 % of a yeast extract (a product the Kyokuto Seiyaku) each charged into 5 Erlenmeyer flasks of 500 ml capacity and sterilized by heating at 120°C for 20 minutes. 5 ml preliminarily sterilized 10 % octanoly-DL-carnitine (a product of the Sigma) were added to each of the flasks under aseptic conditions, which were then inoculated with a small amount of Alcaligenes sp. No 981. The mixtures were cultivated at 28°C for 72 hours on a shaking cultivator with shaking at 120 r.p.m. A total of 470 ml of the culture product liquor was obtained.

### Example 2 Separation and Purification of the Acylcarnitine Esterase According to the Invention

470 ml of the culture product liquor were subjected to centrifugation to collect the bacterial cells, which were suspended in 50 ml of 100 mM tris buffer solution (pH 8.0). This suspension was homogenized using an ultrasonic disintegrator (made by Kubota), whereupon it was subjected to cenrifugation at 15,000 r.p.m. for 10 minutes to obtain 45 ml of a supernatant (0.3 U.ml). This crude enzyme solution was heat treated at 60 °C for 30 minutes, whereupon it was again centrifuged at 15,000 r.p.m. to obtain 44 ml of a supernantant (0.3 U/ml). The resulting enzyme solution was passed through a gel filtration colomn charged with 20 ml of DEAE-Sepharose CL-6B (made by Pharmacia) which had been buffered with 50 mM tris-HCl buffer solution (pH 8.0), whereupon 100 ml of 5 mM tris-buffer solution (pH 8.0) containing 0.1 M KCl were passed through the thus charged column, before the column was eluted with 50 mM Tris-HCl buffer solution (pH 8.0) containing 0.25 M KCl. By this elution, 25 ml of an enzyme solution (0.47 U/ml) were obtained. This enzyme solution was dialyzed overnight at 5 °C against liters of 50 mM tris-HCl buffer solution (pH 8.0), whereby 28 ml of an enzyme solution (0.41 U/ml; yield = 85 %) were obtained. 20 ml of this enzyme solution were freeze-dried, whereby 80 mg of pulverous product (0.1 U/mg) were obtained.

### Reference Example 1

### Culture of Alcaligenes sp. No 981 for the Production of L-carnitine Dehydrogenase

100 ml of liquid culture medium (pH 7.0) containing 3 % of DL-carnitine chloride (a product of Sigma), 0.2 % of KH₂HO₄, 0.4 % of K₂HPO₄, 0.05 % of M_{g}SO₄ · 7H₂O, 0.002 % of FeSO₄ · 7H₂O, 0.001 % of MnSO₄ · nH₂O were charged in 500 Erlenmeyer flask and were sterilized by heating at 120°C for 20 minutes. This culture medium was inoculated with a small amount of Alcaligenes sp. No 981 and the mixture was cultivated on a shaking cultivator at a shaking rate of 120 r.p.m. at 28 °C for 40 hours to obtain 95 ml of a culture mother liquor (enzymatic activity = 1.2 U/ml).

On the other hand, 20 liters of liquid culture medium (pH 7.0) containing 3 % of DL-carnitine chloride (a product of Sigma), 0.1 % of a yeast extract (a product of Kyokuto Seiyaku), 0.054 % of KH₂HO₄, 0.746 % of K₂HPO₄, 0.05 % of MgSO₄ · 7H₂O, 0.002 % of CaCl₂ · 2H₂O, 0.002 % of FeSO₄ · 7H₂O, 0,002 % of MnSO₄ · nH₂O and 1 ml/l of Disfoam CB442 (a product of Nippon Oils and Fats) were charged in a 30 l jar fermenter and were sterilized by heating. This liquid culture medium was inoculated with 90 ml of the culture mother liquor obtained above and the mixture was subjected to aeration cultivation under conditions of a culture temperature of 28°C, an aeration rate of 20 liter/ min., a fermenter inner pressure of 0.4 kg/cm, an agitation rate of 200 r.p.m. and a culture duration of 27 hours, whereby 19 litters of culture product (enzymatic activity = 3.0 U/ml) were obtained.

### Reference Example 2

### Separation and Purification of L-carnitine Dehydrogenase

90 liters of the culture product obtained in Reference Example 1 were subjected to centrifugation for collecting the bacterial cells. The collected bacterial cells were subjected to a solubilization treatment at 37°C for 1 hour by adding thereto 4 liters of a 40 mM tris-HCl buffer solution (pH 8.0) containing 0.1 % of lysozyme and 15 mM of ethylenediamine tetraacetic acid disodium salt (EDTA · 2Na). The treated mixture was subjected to centrifugation to obtain 4,500 ml of a supernatant (having a specific enzymatic activity of 10.3 U/ml). this supernantant, 1,100 g of ammonium sulfate were dissolved and the thereby formed precipitae was removed by centrifugation, whereupon 700 g of ammonium sulfate were again dissolved in the resulting supernatant. The treated supernatant was then subjected to centrifugation to obtain a precipitate, which was dissolved in 500 ml of 40 mM tris-HCl buffer solution (pH 8.0) and this solution (which has a specific enzymatic activity of 84.1 U/ml) was dialyzed against 10 liters of 40 mM tris-HCl buffer solution (pH 8.0). The dialyzed enzyme solution was passed through a gel filtration column packed with 200 ml of DEAE-Sepharose CL-6B (a product of Pharmacia) which had been buffered with 40 mM tris-HCl buffer solution (pH 8.0), followed by a displacement of the filter bed by passing 1 liter of a 40 mM tris-HCl solution (pH 8.0) containing 0.1 M KCl, whereupon the column was eluted using 40 mM tris-HCl buffer solution (pH 8.0) containing 0.3 M KCl to obtain 300 ml of an enzyme solution (having a specific enzymatic activity of 12.5 U/ml). This enzyme solution was dialyzed against 10 liters of 40 mM tris-HCl buffer solution (pH 8.0). The dialyzed enzyme solution was passed through a column packed with 100 ml of hydroxylapate (a product of KOKEN ) which had been buffered with 40 mM tris-HCl buffer solution followed by a displacement procedure by passing therethrough 200 ml of 40 mM tris-HCl buffer solution (pH 8.0), whereupon the column was eluted with 800 ml of 2 mM phosphate buffer solution (pH 7.0) to obtain 100 ml of an enzyme solution (specifc enzymatic activity = 331 U/ml). This enzyme solution was dialyzed against 5 liters of phosphate buffer solution (pH 7.5), whereby 95 ml of a dialyzed enzyme solution was obtained (specifc enzymatic activity = 331 U/ml; recovery yield = 67.8 %).

The NADH-oxidase activity in this purified L-carnitine dehydrogease was found to be not higher than 0.0001 U/ml.

The properties of the L-carnitine dehydrogenase obtained above were:

### (1) Enzymatic activity

It catalyzes at least the reaction of L-carnitine with NAD to form 3-deydrocarnitine and NADH, as illustrated in the following reaction scheme;
L-carnitine 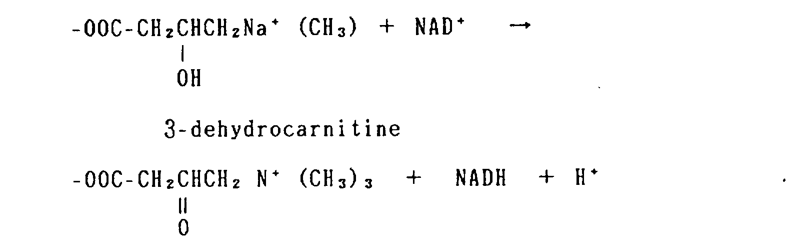 3-dehydrocarnitine

### (2) Substrate-specificity

| Substrate | Relative specif. % |
|---|---|
| L-carnitine | 100 |
| Choline | 0 |
| Glycinebetaine | 0 |
| Glucose | 0 |
| Lysine | 0 |

### (3) Molecular weight

$\text{51,000 ± 6,000}$

The molecular weight was determined by molecular sieve chromatography using TSK Gel G3000 SW (a product of Toso ) on a column of 7.5 × 60 cm using an eluent consisting of a 0.1 M phosphate buffer solution (pH 7.0) containing 0.2 M NaCl using the following standard molecular weight markers supplied from Oriental Kobo :

| Molecular Weight | Compound |
|---|---|
| 12,000 | Cytochrome C |
| 32,000 | Adenilate Kinase |
| 67,000 | Enolase |
| 142,000 | Lactate dehydrogenase |
| 290,000 | Glutamate dehydrogenase |

### (4) Isoelectric point

$\text{pH 5.3 ± 0.6}$

The isoelectric point was determined by isoelectric focussing using a carrier ampholite under a constant voltage of 700 V for 40 hours, whereupon the solution was subjected to a fractionation and each fraction was examined for enzymatic activity.

### (5) Km value

The Km value for NAD⁺ was determined by varying the concentration of the NAD⁺ in a reaction solution containing 100 mM tris-HCl buffer solution (pH 9.0), 5 Unites of diaphorase (a product of Toyo Jozo), 0.025 % of NBT (a product of Wako Pure Chemical Ind.), 1 % of Tween 80 (a product of Wako Pure Chemical Ind.) and 50 mM L-carnitine, whereby a value of 0.141 mM was obtained.

On the other hand, the Km value for L-carnitine was determined by varying the concentration of L-carnitine in the above reaction solution in which 50 mM of L-carnitine was replaced by 1 mM NAD⁺ , whereby a value of 9.3 mM obtained.

### (6) Heat stability

A solution of this esterase (1.00 U/ml) was prepared using 20 mM tris-HCl buffer solution (pH 8.0). This solution was heat treated for 1 hour, whereupon the residual enzymatic activity was determined in accordance with the method for determining enzymatic activity described below, which showed that the enzymatic activity was stable at least up to a temperature of 45°C .

### (7) Optimum temperature

Using 100 mM tris-HCl buffer solution (pH 9.0), storage stability at 5 °C for 2 weeks was observed. The L-carnitine dehydrogenases obtained from the three strains of L-carnitine dehydrogenase-producing known bacteria cited previously showed to have residual enzymatic activities after one week of about 53 - 40 %, which was decreased to below 45°C after two weeks. In particular, the enzyme produced from Pesudomonas aeruginosa IFO 13130 was at the most unstable and exhibited a residual activity of only 41 %. In contrast thereto, the L-carnitine dehydrogenase obtained from the bacterium according to the present invention exhibited a residual enzymatic activity of 96 % after one week and 82 % after two weeks, showing that it has considerably higher stability as compared with the conventional enzymes derived from known L-carnitine dehydrogenase-producing bacteria. When 0.05 mM NAD ⁺ was caused to coexist in this storage stability test, the residual enzymatic activity was found to be 99.7 % after one week and 95.1 % after two weeks, which is even higher than the above, suggesting a stabilizing effect of NAD⁺ .

### (8) Method for determining the enzymatic activity of the L-carnitine dehydrogenase

### ① Composition of the reaction solution

| | |
|---|---|
| Tris-HCl buffer solution (pH 8.0) | 50 mM |
| NAD⁺ | 1 mM |
| Diaphorase (of Toyo Jozo) | 5 Units |
| NBT (of Wako Pure Chemical Ind.) | 0.025 % |
| KCl | 100 mM |
| Polyoxyethylene (20) sorbitan monooleate (of Wako Pure Chemical Ind.) | 0.5 % |
| L-carnitine (of Sigma) | 100 mM |

### ② Determination of Enzymatic activity

1 ml of the reaction solution was placed in a small test tube and s incubated at 37 °C for 5 minutes, and 0.02 ml of the esterase solution to be examined is then added to start the reaction with agitation. After exactly 10 minutes, 2 ml of 0.1 N aq. HCl are added thereto to terminate the reaction and A_{550 nm} of the solution is measured to obtain the absorbance A₁.

The same procedures are repeated for the reaction solution in which L-carnitine has been removed, to obtain the absorbance Aₒ .

### ③ Calculation equation

$\text{U/ml=} \frac{{\text{(A}}_{\text{1}} {\text{-A}}_{\text{2}} \text{)}}{\text{21.7}} \text{×} \frac{\text{1}}{\text{10}} \text{×} \frac{\text{3.02}}{\text{0.02}} \text{×Z}$ (in which Z denotes the dilution magnification ratio and the numeral 21.7 corresponds to the molecular extinction coefficient in cm/ µmol)

### Example 3

### Comparison of Hydrolysis Performance of Octanoyl-L-carnitine by the Acylcarnitine Esterase of the Invention with that by aq. Potassium Hydroxide

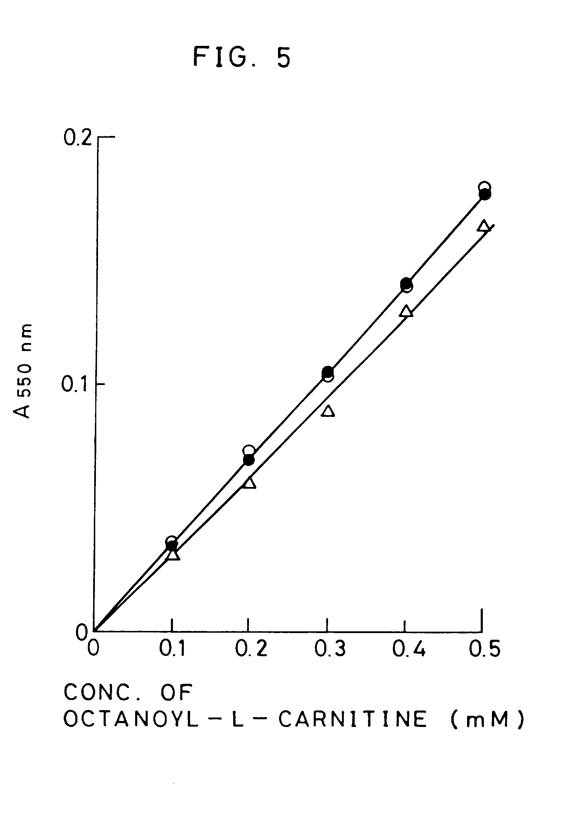

Sample solutions of octanoyl-L-carnitine were prepared using 50 mM tris-HCl buffer solution (pH 8.0) in such a way that the octanoyl-L-carnitine concentrations were 0.1 mM, 0.2 mM, 0.3 mM, 0.4 mM and 0.5 mM respectively. Each 1 ml of these solutions was charged in a small test tube incubated at 37°C for 5 minutes, 0.1 ml of the enzyme solution (0.41 U/ml) prepared in Example 2 was added and the resulting solution was further incubated for 15 minutes. Each 0.05 ml of these enzyme solution-added solution was then added to each 1 ml of separately prepared reaction solutions of L-carnitine of analysis method A which had each been preliminarily inoculated in a small test tube 37°C. Each of the treated solutions was incubated for 2 minutes and, then, 2 ml of 0.1 N aq. HCl was added and the resulting solution was examined at A₅₅₀ nm . Parallel thereto, sample solutions were prepared similarly but using KOH solution as the hydrolyzing agent. Thus, 1 ml of 10 N KOH solution was added to 1 ml of octanoyl-L-carnitine and the resulting solution was incubated at 37°C for 3 hours, whereupon the solution was neutralized by adding 1 ml of 10 N aq. HCl. 0.15 ml of this neutralized solution was added to 1 ml of the reaction solution of the analysis method A [cf. ④ of para. 10 Method for the estimating the activity of acylcarnitine esterase] , whereupon the resulting solution was examined at A_{550 nm} in the same manner. The results are given in the graph of Fig. 5, which shows that octanoyl-L-carnitine had been completely hydrolyzed by the enzyme according to the present invention. In Fig. 5, the marks ○, Δ and ● stands for the values obtained by the acylcarnitine esterase according to the present invention, that obtained by KOH and that calculated theoretically, respectively.

### Example 4

### Determination of Acyl-L-carnitine in Human Serum

The amount of free L-carnitine in a male human serum was determined by the analysis method A for 5 normal healthy male donors. 1 ml of 10 N aq. potassium hydroxide was added to 1 ml of the serum and the mixture was incubated at 37°C for 2 hours, whereupon this alkali-treated serum was neutralized by adding thereto 1 ml of 10 N aq. HCl. The treated serum was examined by the analysis method A for its total carnitine content. On the other hand, 1 mg (0.1 U/mg) of acylcarnitine esterase according to the present invention was added to 1 ml of fresh serum and this esterase-inoculated serum was incubated at 37 °C for 15 minutes. This esterase-treated serum was also examined by the analysis method A for its total carnitine content. The results are summarized in Table 1. Table 1 shows that acyl-L-carnitines had been hydrolyzed by the esterase according to the present invention as well as by the alkali.

**Table 1**

| Serum Sample (Donor Age) | Conc. of Free L-carnitine detected (in µM) | Total L-carnitine Conc. detected (in µM) | |
|---|---|---|---|
| | | Hydrolyzed by | |
| | | Esterase | KOH |
| 1 (20) | 40.7 | 71.9 | 70.8 |
| 2 (23) | 45.3 | 73.2 | 74.9 |
| 3 (34) | 37.6 | 69.8 | 67.0 |
| 4 (37) | 44.0 | 65.1 | 65.7 |
| 5 (48) | 42.9 | 71.5 | 70.3 |

### Reference Example 3

### Purification of Acylcarnitine Esterase Originated from the Liver of a Rat

For comparison of the esterase performance with that of the esterase according to the present invention, acylcarnitine esterase was collected from the liver of a rat and purified according to the technique described by S. Mahadevan and F. Sauer given in "J. Biol. Chem.,", 244, No. 16, 4448 - 4453 (1969). From the homogenate of 50 g of the rat liver, acylcarnitine esterase was extracted and purified using DEAE-cellulose (Whatman DE-52) and Sephadex G-200 (supplied from of Pharmacia), whereby 101.6 mg (0.0364U/ml) of a freeze-dried product were obtained.

### Reference Example 4

Using the esterase produced in Reference Example 3, the enzymatic activity and Km values for acetyl-caritine, propionyl-L-carnitine, octanoyl-L-carnitine, decanoyl-L-carnitine and palmitoyl-L-carnitne were determined. The results are given in Table 2. The esterase does not exhibit enzymatic activity for the short chain acyl-L-carnitine, i.e. acetyl-L-carnitine and propionyl-L-carnitine, while it does exhibit enzymatic activity for other medium to long chain acyl-L-carnitines. The Km values for these acyl-L-carnitines were found to be considerably high, i. e. of the order of 10 ⁻³ M as reported in the literature.

**Table 2**

| Acyl-L-carnitine | Enzymatic Activity (rel. %) | Km Value (× 10⁻³) |
|---|---|---|
| Acetyl-L-carnitine | 0 | - |
| Propionyl-L-carnitine | 0 | - |
| Octanoyl-L-carnitine | 44 | ca. 3 |
| Decanoyl-L-carnitine | 100 | ca. 2 |
| Palmitoyl-L-carnitine | 27 | ca. 5 |

### Example 5

The esterase prepared in Reference Example 3 was examined for its heat stability.

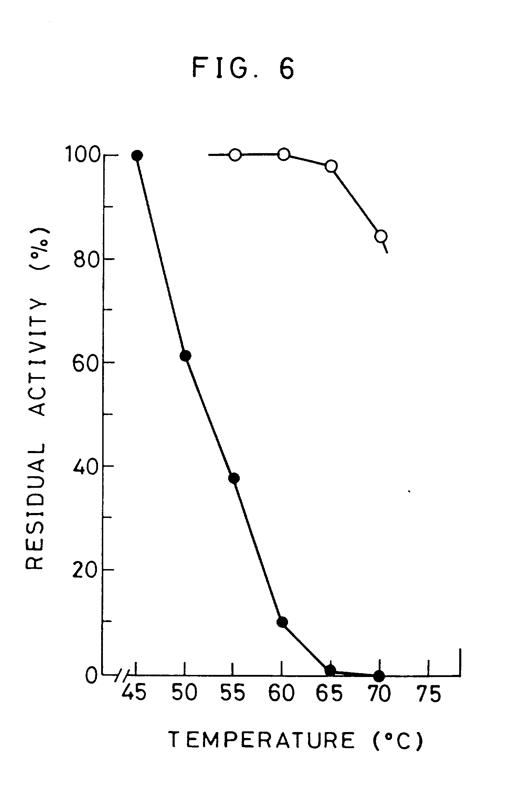

Sample solutions were prepared using 100 mM tris-HCl buffer solution (pH 8.0) so that they all had an enzyme concnetration of 0.1 U/ml. Using these sample solutions, the residual enzymatic activity remaining after heat treatment at 45°C, 50°C, 55°C , 60°C, 65°C or 70 °C each for 30 minutes was determined. The results are given in the graph of Fig. 6, from which it is seen that the enzymatic activity decreased to 61 % at a temperature of 50 °C. In the graph, the ploted marks ● and ○ correspond to the data for rat-originated esterase and to that for the esterase according to the present invention respectively.

### Example 6

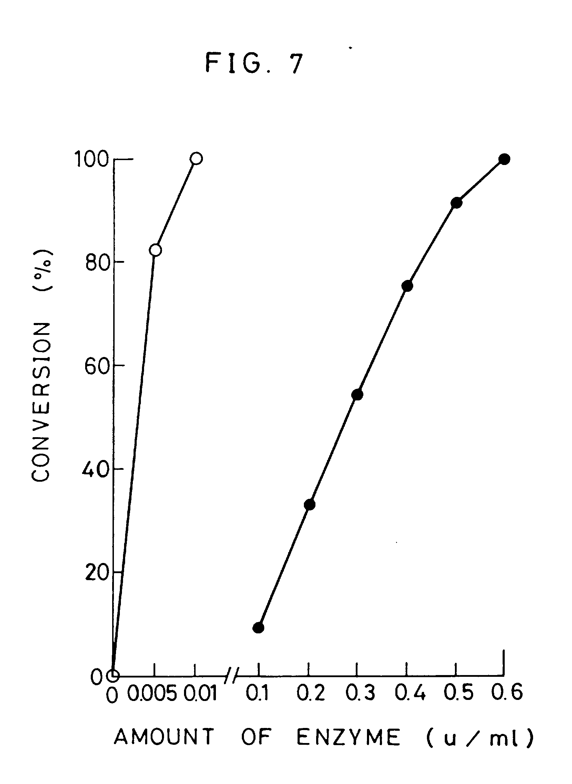

1ml of the L-carnitine sample solution prepared from 100 mM tris-HCl solution (pH 8.0) containing 0.02 mM octanoyl-L-carnitine with varying amounts of esterase originated from the liver of a rat prepared in Reference Example 3 and with varying amount of esterase according to the present invention prepared in Example 2 was subjected to enzymatic hydrolysis at 37°C for 50 minutes and the amount of L-carnitine formed was determined. The results are given in the graph of Fig. 7, which shows that the enzymatic hydrolysis had almost been completed at an esterase content of 0.01 U/ml for the esterase according to the present invention, while the enzymatic reaction was not completed for the esterase originated from the liver of a rat even at an esterase content of 0.6 U/ml.

### Example 7

Using the purified esterase originated from the liver of a rat and that according to the invention, analysis of acyl-L-carnitine was carried out. To 1 ml of each of the solutions prepared from 100 mM tris-Hcl buffer solution (pH 8.0) each containing 1 mM of acetyl-L-carnitine, propionyl-L-carniitne, hexanoyl-L-carnitine, octanoyl-L-carnitine, decanoyl-L-carnitine, lauroyl-L-carnitine, myristoyl-L-carnitine, palmitoyl-L-carnitne and stearoyl-L-carnitine, there was added 0.1 U (1 mg) of the esterase according to the present invention. The resulting solution was incubated at 37 °C for 30 minutes. On other hand, 1 Unit (27.5 mg) of purified esterase originated from the liver of a rat was added to 1 ml of the same acyl-L-carnitine-buffer solution and the solution was also incubated at 37°C for 30 minutes. To 1 ml of the reaction solution of the analysis method A for determining L-carnitine which had been incubated preliminarily at 37°C for 5 minutes, there was added 0.01 ml of one of either of the reacted esterase solutions prepared as above and the resulting mixture was incubated at 37 °C for two minutes. Thereto were then added 2 ml of 0.1 Naq. HCl and A_{550 nm} was then measured in order to determine the amount of L-carnitine produced. On the other hand, one reaction solution to which 0.01 ml of the rat-originated esterase solution had been added and incubated was further treated by adding 0.1 Unit of the esterase according to the present invention and further incubated at 37°C for 30 minutes, whereupon this supplementarily treated solution was examined for A_{550 nm} . The results are summarized in Table 3, from which it is shown that in the sample solutions treated with the esterase according to the present invention, all the acyl-L-carnitine had completely been hydrolyzed, whereas in the sample solutions treated with the esterase originated from the liver of a rat, only the acyl-L-carnitines other than the short chain acyl-L-carnitines, namely, acetyl-L-carnitine and propionyl-L-carnitine, had been hydrolyzed. By supplementarily treating further with the esterase according to the present invention, the short chain acyl-L-carnitines are hydrolyzed.

**Table 3**

| Treated by | A_{550 nm} observed | Acyl-L-carnitines determined |
|---|---|---|
| Theoretically | 0.649 | 9.0 mM (100 %) |
| Inventive esterase | 0.662 | 9.18 mM (102 %) |
| Rat-derived esterase | 0.487 | 6.76 mM ( 75 %) |
| Rat-derived esterase + inventive estearse | 0.636 | 8.82 mM (98 %) |

### Example 8

To 1 ml of serum of a normal healthy male human donor (Serum Sample 1 in Table 1 of Example 4), 1 Unit (27.5 mg) of purified esterase originated from the liver of a rat was added and the mixture was incubated at 37°C for 30 minutes, whereby enzymatically hydrolyzed Reacted Liqour 1 was obtained. 0.1 ml of Reacted Liqour 1 was added to 1 ml of the reaction solution of the analysis method A for determining L-carnitine which had been incubated preliminarily at 37°C for 5 minutes, and the resulting solution was incubated at 37°C for 5 minutes. This incubated solution was then examined at A_{550 nm}, whereby an A_{550 nm} value of 0.121 was obtained. On the other hand, a Reacted Liquor 2 was prepared by adding to 0.9 ml of Reacted Liquor 1 0.09 Unit (0.9 mg) of the esterase according to the present invention and incubating the resulting mixture at 37 °C for 30 minutes. Reacted Liqour 2 was also examined at A_{550 nm} in the same manner as that for Reacted Liquor 1, whereby an A_{550 nm} value of 0.142 was obtained.

From the above experimental results, the carnitine profile in the serum of male donor 1 is estimated to be as given in the following Table 4.

**Table 4**

| Carnitines | concentration |
|---|---|
| Free L-carnitine | 40.7 µM |
| Total carnitine | 71.9 µM |
| Acyl-L-carnitine | 31.2 µM |
| Medium-long chain acyl-L-carnitines | 20.6 µM |
| Acetyl-and propionyl-L-carnitine | 10.6 µM |

### Example 9

### Reaction solution composition

| | | |
|---|---|---|
| (Ia) | 20 mM | tris-HCl buffer solution (pH 8.0) |
| | 0.2 % | Polyoxyethylene (20) sorbitan monooleate (Wako Pure Chem. Ind.) |
| (Ib) | 20 mM | tris-HCl buffer solution (pH 8.0) |
| | 0.2 % | Polyoxyethylene (20) sorbitan monooleate (Wako Pure Chem. Ind.) |
| | 0.2 U/ml | Acyl-L-carnitine esterase (from Alcaligenes sp. No. 981 supplied from Toyo Jozo) |

| | | |
|---|---|---|
| (Ic) | 20 mM | tris-HCl buffer solution (pH 8.0) |
| | 0.2 % | Polyoxyethylene (20) sorbitan monooleate (Wako Pure Chem. Ind.) |
| | 1 U/ml | Acyl-L-carnitine esterase (originated from liver of rat) |

| | | |
|---|---|---|
| (II) | 200 mM | tris-HCl buffer solution (pH 9.5) |
| | 8 mg | Thio-NAD (supplied from Sigma) |
| | 0.2 mM | NADH (supplied from Oriental Kobo) |
| | 370 U/ml | L-carnitine dehydrogenase (from Alcaligenes sp. No. 981 supplied from Toyo Jozo) |

To 25 ml of the serum of male donor 1, 0.5 ml of the above solution (Ia) and 0.5 ml of the above solution (II), both of which had been incubated preliminarily at 37 °C, were added and the mixture was warmed at 37°C . The difference between the data of absorbance at 400 nm determined after 3 minutes and after 5 minutes from the addition of the solution was calculated [A _{(mAbs)} ] . The same procedures were applied also for the solution (Ib) to calculate the difference of the absorbance data (B). Furthermore, the same procedure were applied also for distilled water, for the standard 50 µ M L-carnitine solution and for the solution (Ia) alone respectively without using serum, whereby R_{B} and S were obtained.

From these results, the content of free L-carnitine and total carnitine content are calculated by the following equations:$\text{(1) Free L-carnitine (µM) =} \frac{{\text{(B-R}}_{\text{B}} \text{)}}{{\text{(S-R}}_{\text{B}} \text{)}} \text{×50}$$\text{(2) Total carnitine (µM) =} \frac{{\text{(A-R}}_{\text{B}} \text{)}}{{\text{(S-R}}_{\text{B}} \text{)}} \text{×50}$

Separately , 25 ml of the above-mentioned serum were added to 0.5 ml of the above solution (Ic) containing acyl-L-carnitine esterase originated from the liver of a rat and the resulting mixture was warmed at 37 °C for 30 minutes, in order to cause all the acyl-L-carnitines other than acetyl-L-carnitine and propionyl-L-carnitine to be hydrolyzed. To the treated mixture, 0.5 ml of the above solution (II) was added and the resulting mixture was warmed at 37 °C, whereupon the absorbance at 400 nm was observed after 3 minutes and after 5 minutes from the addition of the solution (II), in order to obtain the difference between them by calculation. The total carnitine content except acetyl- and propionyl-L-carnitine is calculated from the following equation: .$\text{(3) Total carnitine without acetyl- and propionyl-L-carnitine (µM) =} \frac{{\text{(B-R}}_{\text{B}} \text{)}}{{\text{(S-R}}_{\text{B}} \text{)}} \text{×50}$

The content of acetyl-L-carnitine plus propionyl-L-carnitine is calculated from the equation$\text{(4) Acetyl- + propionyl- = (2) - (3)}$ L-carnitne (µM)

These results are summarized in the following Table. As can be seen, the serum carnitine profile of Sample 1 coincides nearly with the results obtained in Example 8.

### Difference of Absorbances at 400 nm at Occasions of 3 and 5 minutes from start

| A | B | C | S | R_{B} |
|---|---|---|---|---|
| 29 mAbs | 49 mAbs | 41 mAbs | 35 mAbs | 2 mAbs |

| Total carnitine | Acyl-L-carnitine | | Free L-carnitine |
|---|---|---|---|
| | Acetyl- and propionyl-L-carnitine | Others | |
| 71.2 µM (2) | 30.3 µM | | 40.9 µM (1) |
| | 12.1 µM (4) | 18.2 µM | |
| | | 59.1 µM (3) | |

### Example 10

Reaction mixture

| | |
|---|---|
| 40 mM | tris-HCl buffer solution (pH 8.0) |
| 0.5 % | Polyoxyethylene (20) sorbitan monooleate (Wako Pure Chem. Ind.) |
| 5 mM | NAD ⁺ |
| 50 U | L-carnitine dehydrogenase (from Alcaligenes sp. No. 981 supplied from Toyo Jozo) |
| 0.1 mM | Acetyl-L-carnitine |
| 0.1 mM | Octanoyl-L-carnitine |

### Operation and Results

1 ml of the above reaction solution was placed in a small test tube and incubated at 37 °C for 5 minutes. There to was added 1 Unit (27.5 mg) of the purified acylcarnitine esterase originated from the liver of a rat and the resulting solution was again incubated at 37°C for 30 minutes. This provided an A_{340 nm} value of 0.561. To this solution, 0.025 ml of 5 N aq. HCl was added and the resulting acidified solution was incubated at 37°C for 15 minutes, whereupon the solution was neutralized by adding 0.025 ml of 5 N aq. KOH .The neutralized solution provided an A_{340 nm} value of 0.08. To the treated reaction solution, 0.01 ml of 500 mM NAD⁺ , 0.01 ml of L-carnitine dehydrogenase and 0.1 Unit (1 mg) of the esterase according to the present invention were further added and the resulting mixture was again incubated at 37°C for 30 minutes. The resulting solution provided a A_{340 nm} value of 0.594.

As described above, all the acetyl-L-carnitnes in the sample solution were able to be determined by first subjecting the sample solution to an enzymatic hydrolysis with the esterase originated from the liver of a rat in order to hydrolyze octanoyl-L-carnitine, then, treating the L-carnitine formed thereby with the L-carnitine dehydrogenase and NAD⁺ to convert the L-carnitine into dehydrocarnitine with conversion of NAD into NADH and, thereafter, removing the formed NADH by acid decomposition by addition of hydrochloric acid and, finally, treating the resulting reaction mixture by renewed addition of the L-carnitne dehydrogenase together with NAD ⁺ and the esterase according to the present invention.

### Example 11

Reaction mixture

| | |
|---|---|
| 50 mM | Glycine-NaOH (pH 10.0) |
| 5 mM | thio-NAD (supplied from Sigma) |
| 30 U/ml | L-carnitine dehydrogenase (from Alcaligenes sp. No. 981 supplied from Toyo Jozo) |
| 0.5 % | Polyoxyethylene (20) sorbitan monooleate (Wako Pure Chem. Ind.) |
| 0.1 M | Sodium chloride |

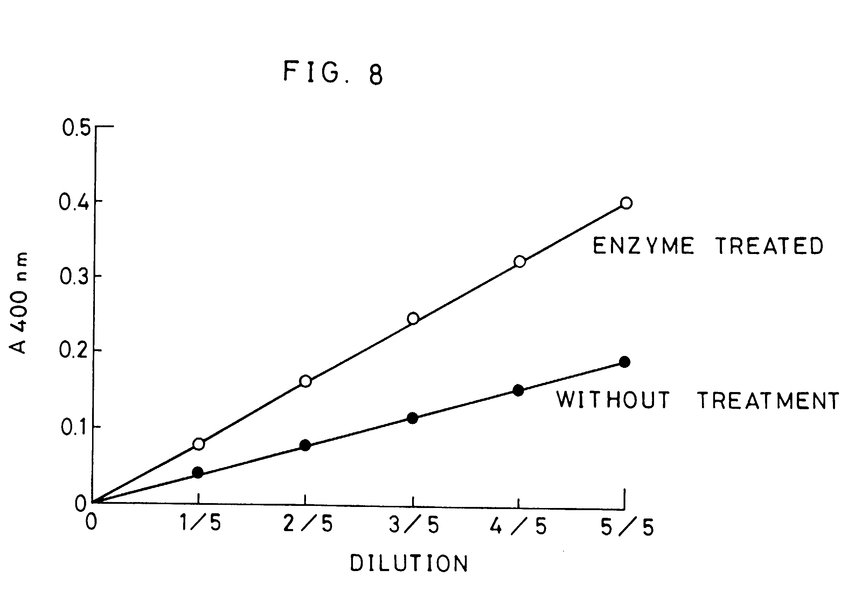

A mixed solution consisting of 250 µ M of L-carnitine and 250 µ M of acetyl-L-carnitine was prepared using 50 mM tris-HCl buffer solution (pH 8.0). This mixed solution was diluted by 5 different stepwise dilution ratios using the same 50 mM tris-HCl buffer solution (pH 8.0) to obtain 5 sample solutions each having gradationally different concentrations. Each of these 5 samples was taken in two test tubes each in an amount of 0.5 ml and the test tubes were warmed preliminarily to 37 °C. To one test tube was added 0.05 ml of acylcarnitine esterase solution (0.41 U/ml) of Example 2 (to serve as an enzyme treated sample) and to the other test tube was added 0.05 ml of distilled water (to serve as a sample without enzyme treatment), whereupon both were warmed at 37 °C for 15 minutes. To 1 ml of the above reaction solution which had preliminarily been warmed at 37°C, there was added each 0.1 ml of either the above enzyme treated sample solution (indicated in the graph of Fig. 8 by the mark o) or the above sample solution without enzyme treatment (indicated in the graph of Fig. 8 by the mark ●), whereupon the resulting solution was warmed at 37 °C for 10 minutes. The treated solutions were then observed to measure the absorbance at 400 nm.

For the blank test, 15 mM tris-HCl buffer solution (pH 8.0) was employed in place of the sample solution. In the graph in Fig 8, values in which the blank test values have already been subtracted from the observed values are plotted.

## Claims

1. An acylcarnitine esterase which:
(a) is specific to each of the following substrates:
acetyl-L-carnitine, propionyl-L-carnitine and palmitoyl-L-carnitine;
(b) catalyzes the hydrolysis of one mole of a said substrate with one mole of water to form one mole of the corresponding acid and one mole of L-carnitine; and
(c) has the following physicochemical properties:
a) molecular weight of 63,000 ± 7,000 (as determined by gel filtration);
b) isoelectric point of pH 5.1± 0.5;
c) Km value for acetyl-L-carnitine of 4 x 10⁻⁵ M, for propionyl-L-carnitine of 3 x 10⁻⁵ M and for palmitoyl-L-carnitine of 2 x 10⁻⁵ M;
d) optimum pH of around pH 8;
e) stability at a pH of from 7.5 to 8.5 at 60°C for 30 minutes; and
f) an optimum temperature of around 70°c at which it exhibits its maximum activity in 100 mM Tris-HCl buffer solution at pH 8.

2. An acylcarnitine esterase according to claim 1 which is obtainable from Alcaligenes sp. No. 981 (FERM BP-2570) or a mutant thereof capable of producing the acylcarnitine esterase.

3. A process for the production of an acylcarnitine esterase as defined in claim 1 or 2, which comprises cultivating an acylcarnitine esterase-producing bacterium of the genus Alcaligenes in a suitable culture medium and collecting the thereby produced acylcarnitine esterase from the culture product mixture.

4. A process according to claim 3, wherein the acylcarnitine esterase-producing bacterium of genus Alcaligenes is Alcaligenes sp. No. 981 (FERM BP-2750) or a mutant thereof capable of producing the acylcarnitine esterase.

5. A method of assaying acyl-L-carnitines including acetyl-L-carnitine and propionyl-L-carnitine in a sample, which comprises subjecting the sample to enzymatic hydrolysis with an acyl-carnitine esterase as defined in claim 1 or 2, and then determining the amount of the fatty acids and/or L-carnitine thus formed.

6. A method according to claim 5, wherein determination of the amount of the resulting fatty acids is carried out by liquid chromatography or gas chromatography.

7. A method according to claim 5, wherein determination of the amount of L-carnitine is carried out by subjecting the enzymatic hydrolysis reaction product to the action of a reagent comprising an L-carnitine dehydrogenase and one of the nicotinamide adenine dinucleotide group compounds (NAD group) or of the thionicotinamde adenine dinucleotide group compounds (thio-NAD group) with, if necessary, an addition of a non-ionic detergent, and then determining the amount of the resultant reduced NAD group or thio-NAD group compounds.

8. A method according to any one of claims 5 to 7, wherein the enzymatic hydrolysis is carried out with the L-carnitine dehydrogenase and a combination of either (1) one of the NAD group and one of the reduced thio-NAD group compounds or (2) one of the thio-NAD group and one of the reduced NAD group compounds, to effect the cycling reaction: 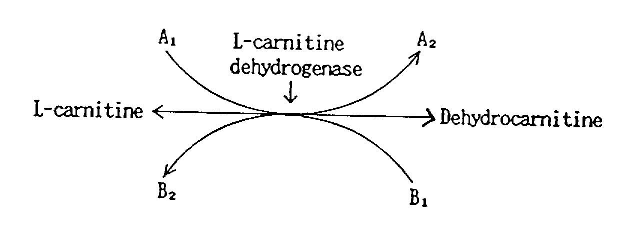 wherein:
A₁ is a NAD group or thio-NAD group compound,
A₂ is the corresponding reduced form of A₁,
B₁ is a reduced thio-NAD group compound when A₁ is a NAD group compound, or is a reduced NAD group compound when A₁ is a thio-NAD group compound, and
B₂ is the corresponding oxidized form of B₁; and the amount of B₁ consumed during the cycling reaction and/or the amount of A₂ formed during the cycling reaction is determined.

9. A method of assaying acetyl-L-carnitine and propionyl-L-carnitine in a sample, which comprises:
(a) subjecting part of the sample to enzymatic hydrolysis with an acylcarnitine esterase which has no enzymatic activity for acetyl-L-carnitine and propionyl-L-carnitine but which exhibits substrate-specificity to acyl-L-carnitines of longer chain lengths and which catalyzes the hydrolysis reaction of one mole of acyl-L-carnitine of said longer chain lengths with one mole of water to form one mole of the corresponding fatty acid and one mole of L-carnitine and then determining the amount of the fatty acids and/or L-carnitine thus formed;
(b) subjecting another part of the sample to an assay of acyl-L-carnitines including acetyl-L-carnitine and propionyl-L-carnitine as defined in any one of claims 5 to 8; and
(c) determining the difference in the analytical values obtained in steps (a) and (b).

10. A method according to claim 9, wherein the acylcarnitine esterase which has no enzymatic activity for acetyl-L-carnitine and propionyl-L-carnitine but which exhibits substrate-specificity to acyl-L-carnitines of longer chain lengths has been obtained from a rat.

11. A method of assaying acetyl-L-carnitine and propionyl-L-carnitine in a sample, which comprises:
(a₁) subjecting the sample to enzymatic hydrolysis with an acylcarnitine esterase which has no enzymatic activity for acetyl-L-carnitine and propionyl-L-carnitine but which exhibits substrate-specificity to acyl-L-carnitines of longer chain lengths and which catalyzes the hydrolysis reaction of one mole of acyl-L-carnitine of longer chain lengths with one mole of water to form one mole of the corresponding fatty acid and one mole of L-carnitine and then subjecting the resultant reacted sample mixture to the action of an L-carnitine dehydrogenase with a coenzyme A₁ (wherein A₁ is a NAD or thio-NAD group compound) to convert A₁ into its reduced form A₂, and treating the resultant sample mixture to decompose A₂; and
(b₁) subjecting the resultant sample mixture to a method of assaying acyl-L-carnitines including acetyl-L-carnitine and propionyl-L-carnitine as defined in any one of claims 5 to 8.

12. Alcaligenes sp. No. 981 (FERM BP-2570) or a mutant thereof which is capable of producing an acylcarnitine esterase as defined in claim 1.

13. A culture of Alcaligenes sp. No. 981 (FERM BP-2570) or a mutant thereof which is capable of producing an acylcarnitine esterase as defined in claim 1, in a culture medium which comprises a source of assimilable carbon, a source of assimilable nitrogen and mineral salts and which is free of other microorganisms.

## Patentansprüche

1. Acylcarnitin-esterase, die
(a) für jedes der folgenden Substrate spezifisch ist: Acetyl-L-carnitin, Propionyl-L-carnitin und Palmitoyl-L-carnitin;
(b) die Hydrolyse von einem Mol der genannten Substrate mit einem Mol Wasser unter Bildung eines Mols der korrespondierenden Säure mit einem Mol L-Carnitin katalysiert; und
(c) die folgenden physikochemischen Eigenschaften besitzt:
a) ein Molekulargewicht von 63.000 ± 7.000 (bestimmt durch Gel-filtration) ;
b) einen isoelektrischen Punkt beim pH 5,1 ± 0,5;
c) einen Km-Wert für Acetyl-L-Carnitidin von 4 x 10⁻⁵ M, für Propionyl-L-Carnitidin von 3 x 10^{-5 M} und für Palmitoyl-L-Carnitidin von 2 x 10⁻⁵ M;
d) ein pH-Optimum bei etwa pH 8;
e) eine Stabilität bei einem pH von 7,5 bis 8,5 bei 60 °C für 30 min.; und
f) ein Temperaturoptimum bei etwa 70 °C, bei der die Acylcarnitin-esterase ihr Aktiviätsmaximum in einer 100 mM Tris-HCl-Pufferlösung beim pH 8 zeigt.

2. Acylcarnitin-esterase nach Anspruch 1, die aus *Alcaligenes* sp. Nummer 981 (FERM BP-2570) oder einer ihrer Mutanten erhältlich ist, die Acylcarnitin-esterase bilden kann.

3. Verfahren zur Herstellung eine Acylcarnitin-esterase gemäß Anspruch 1 oder 2, bei dem man ein Acylcarnitin-esterase bildendes Bakterium der Gattung *Alcaligenes* in einem geeigneten Kulturmedium kultiviert und die dabei gebildete Acylcarnitin-esterase vom Gemisch der Kulturprodukte sammelt.

4. Verfahren nach Anspruch 3, bei dem als Acylcarnitin-esterase bildendes Bakterium der Gattung *Alcaligenes Alcaligenes* sp. Nr. 981 (FERM BP-2750) oder eine seiner Mutanten verwendet, die Acylcarnitin-esterase bilden kann.

5. Verfahren zum Testen von Acyl-L-carnitinen unter Einschluß von Acyl-L-carnitin und Propionyl-L-carnitin in einer Probe, bei dem man die Probe einer enzymatischen Hydrolyse mit einer Acylcarnitin-esterase gemäß Anspruch 1 oder 2 unterwirft und danach die Menge an Fettsäuren und/oder L-Carnitin bestimmt, die auf diese Weise gebildet worden sind.

6. Verfahren nach Anspruch 5, bei dem man die Bestimmung der Mengen an resultierenden Fettsäuren durch Flüssigchromatographie oder Gaschromatograhie durchführt.

7. Verfahren nach Anspruch 5, bei dem man die Bestimmung der Menge an L-Carnitin dadurch durchführt, daß man das Produkt der enzymatischen Hydrolysereaktion der Einwirkung eines Reagens, das eine L-Carnitindehydrogenase und eine Verbindung der Nikotinamidadenindinucleotid-Gruppe (NAD-Gruppe) oder eine Verbindung der Thionikotinamidadenindinucleotid-Gruppe (Thio-NAD-Gruppe) umfaßt, mit gegebenenfalls Zugabe eines nicht-ionischen oberfächenaktiven Mittels unterwirft und danach die Menge der resultierenden reduzierten Verbindungen der NAD-Gruppe oder Thio-NAD-Gruppe bestimmt.

8. Verfahren nach einem der Ansprüche 5 bis 7, bei dem man die enzymatische Hydrolyse mit der L-Carnitindehydrogenase und einer Kombination von entweder
(1) einer Verbindung der NAD-Gruppe und einer reduzierten Verbindung der Thio-NAD-Gruppe oder
(2) eine Verbindung der Thio-NAD-Gruppe und einer reduzierten Verbindung der NAD-Gruppe durchführt und die zyklische Reaktion erreicht: 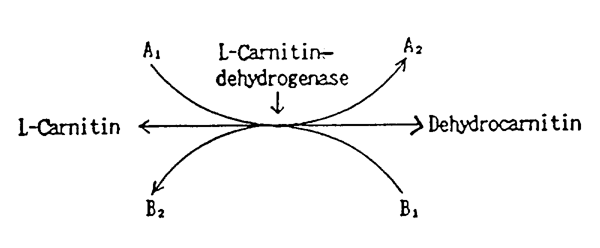 worin:
A₁ eine Verbindung der NAD-Gruppe oder der Thio-NAD-Gruppe ist,
A₂ die korrespondierende reduzierte Form von A₁ ist,
B₁ eine reduzierte Verbindung der Thio-NAD-Gruppe ist, sofern A₁ eine Verbindung der NAD-Gruppe ist, oder eine reduzierte Verbindung der NAD-Gruppe ist, sofern A₁ eine Verbindung der Thio-NAD-Gruppe ist, und
B₂ die korrespondierende oxidierte Form von B₁ ist; und
die Menge an B₁, die während der Zyklisierungsreaktion verbraucht wird, und/oder die Menge an A₂ bestimmt, die während der Zyklisierungsreaktion gebildet wird.

9. Verfahren zum Testen van Acetyl-L-carnitin und Propionyl-L-carnitin in einer Probe, bei dem man
(a) einen Teil der Probe einer enzymatischen Hydrolyse mit einer Acylcarnitin-esterase unterwirft, die keine enzymatische Aktivität für Acetyl-L-carnitin und Propionyl-L-carnitin besitzt, jedoch Substratspezifität für Acyl-L-carnitine mit längeren Ketten zeigt und die Hydrolysereaktion von einem Mol Acyl-L-carnitin mit den längeren Ketten mit einem Mol Wasser unter Bildung von einem Mol der korrespondierenden Fettsäure und einem Mol L-Carnitin katalysiert und danach die Menge an Fettsäuren und/oder an L-Carnitin bestimmt, die auf diese Weise gebildet wurden;
(b) einen weiteren Teil der Probe einem Test auf Acyl-L-carnitine unter Einschluß von Acetyl-L-carnitin und Propionyl-L-carnitin gemäß einem der Ansprüche 5 bis 8 unterwirft; und
(c) die Differenz der analytischen Werte bestimmt, die bei den Stufen (a) und (b) erhalten worden sind.

10. Verfahren nach Anspruch 9, bei dem man eine Acylcarnitin-esterase verwendet, die keine enzymatische Aktivität für Acetyl-L-carnitin und Propionyl-L-carnitin besitzt, jedoch Substratspezifität für Acyl-L-carnitine mit längeren Ketten zeigt und aus Ratten erhalten worden ist.

11. Verfahren zum Testen von Acetyl-L-carnitin und Propionyl-L-carnitin in einer Probe, bei dem man
(a₁) die Probe einer enzymatischen Hydrolyse mit einer Acylcarnitin-esterase unterwirft, die keine enzymatische Aktivität für Acetyl-L-carnitin und Propionyl-L-carnitin besitzt, jedoch Substratspezifität für Acyl-L-carnitine mit längeren Ketten zeigt und die Hydrolysereaktion von einem Mol Acyl-L-carnitin mit längeren Ketten mit einem Mol Wasser unter Bildung von einem Mol der korrespondierenden Fettsäure und einem Mol L-Carnitin katalysiert und danach das resultierende Gemisch der Reaktionsprobe der Einwirkung einer L-Carnitin-dehydrogenase mit einem Coenzym A₁ (worin A₁ eine Verbindung der NAD-Gruppe oder Thio-NAD-Gruppe ist), A₁ in seine reduzierte Form A₂ überführt und das resultierende Gemisch der Probe behandelt und A₂ zersetzt; und
(b₁) das resultierende Gemisch der Probe einem Verfahren zum Testen von Acyl-L-carnitinen unter Einschluß von Acetyl-L-carnitin und Propionyl-L-carnitin gemäß einem der Ansprüche 5 bis 8 unterwirft.

12. *Alcaligenes* sp. Nr. 981 (FERM BP-2570) oder einer seiner Mutanten, die eine Acylcarnitin-esterase gemäß Anspruch 1 bilden kann.

13. Kultur von *Alcaligenes* sp. Nr. 981 (FERM BP-2570) oder einer seiner Mutanten, die Acylcarnitin-esterase gemäß Anspruch 1 in einem Kulturmedium bilden kann, das eine assimilierbare Kohlenstoffquelle, eine assimilierbare Stickstoffquelle und Mineralsalze umfaßt und frei von anderen Mikroorganismen ist.

## Revendications

1. Acylcarnitine estérase qui :
(a) est spécifique à chacun des substrats suivants : acétyl-L-carnitine, propionyl-L-carnitine et palmitoyl-L-carnitine ;
(b) catalyse l'hydrolyse d'une mole d'undit substrat avec une mole d'eau pour former une mole de l'acide correspondant et une mole de L-carnitine ; et
(c) a les propriétés physiques ou chimiques suivantes :
a) poids moléculaire de 63000 ± 7000 (comme déterminé par filtration sur gel) ;
b) point isoélectrique de pH 5,1 ± 0,5 ;
c) valeur Km pour l'acétyl-L-carnitine de 4 x 10⁻⁵ M, pour la propionyl-L-carnitine de 3 x 10⁻⁵ M et pour le palmitoyl-L-carnitine de 2 x 10⁻⁵ M ;
d) pH optimal autour de pH 8 ;
e) stabilité à un pH de 7,5 à 8,5 à 60°C pendant 30 minutes ; et
f) une température optimale d'environ 70°C à laquelle elle montre son activité maximale dans une solution tampon tris-HCl 100 mM à pH 8.

2. Acylcarnitine estérase selon la revendication 1 qui peut être obtenue à partir d'alcaligenes sp. N° 981 (FERM BP-2570) ou son mutant capable de produire l'acylcarnitine estérase.

3. Procédé pour la production d'une acylcarnitine estérase tel que définie en revendication 1 ou 2, qui comprend la culture d'une bactérie produisant de l'acylcarnitine estérase du gène Alcaligenes dans un milieu de culture approprié et la collecte de l'acylcarnitine estérase ainsi produite à partir du milieu du produit de culture.

4. Procédé selon la revendication 3, dans lequel la bactérie produisant l'acylcarnitine estérase du gène Alcaligenes est Alcaligenes sp. N° 981 (FERM BP-2750) ou son mutant capable de produire l'acylcarnitine estérase.

5. Méthode de dosage d'acyl-L-carnitines incluant l'acétyl-L-carnitine et la propionyl-L-carnitine dans un échantillon, qui comprend la soumission de l'échantillon à une hydrolyse enzymatique avec une acyl-carnitine estérase telle que définie en revendications 1 ou 2, et ensuite détermination de la quantité d'acides gras et/ou de L-carnitine ainsi formée.

6. Méthode selon la revendication 5, dans laquelle la détermination de la quantité des acides gras résultants est mise en oeuvre par chromatographie liquide ou chromatographie gazeuse.

7. Méthode selon la revendication 5, dans laquelle la détermination de la quantité de L-carnitine est mise en oeuvre en soumettant le produit de réaction d'hydrolyse enzymatique à l'action d'un réactif comprenant une L-carnitine déhydrogénase et l'un des composé du groupe des nitotinamide adénine dinucléotides (groupe NAD) ou des composés du groupe des thionicotinamide adénine dinucléotides (groupe thio-NAD) avec, si nécessaire, une addition d'un détergent non ionique, et ensuite détermination de la quantité des composés résultants du groupe NAD réduit ou du groupe thio-NAD.

8. Méthode selon l'une quelconque des revendications 5 à 7, dans laquelle l'hydrolyse enzymatique est mise en oeuvre avec la L-carnitine déhydrogénase et une combinaison de soit (1) l'un des composés du groupe NAD et l'un des composés du groupe des thio-NAD réduit ou (2) l'un des composés du groupe des thio-NAD et l'un des composés du groupe des NAD réduits, pour effectuer la réaction de cycle : 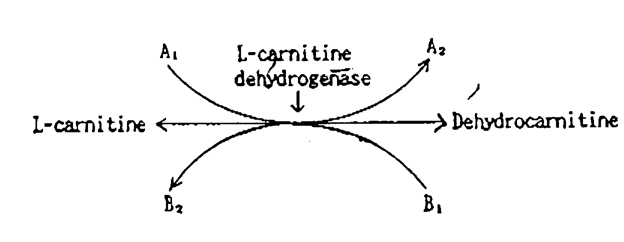 où :
A₁ est un composé du groupe des NAD ou du groupe des thio-NAD,
A₂ est la forme réduite correspondante de A₁,
B₁ est un composé du groupe des thio-NAD réduits lorsque A₁ est un composé du groupe des NAD, ou est un composé du groupe des NAD réduits lorsque A₁ est un composé du groupe des thio-NAD, et
B₂ est la forme oxydée correspondante de B₁ ; et la quantité de B₁ consommée pendant la réaction de cycle et/ou la quantité de A₂ formée durant la réaction de cycle est déterminée.

9. Méthode de dosage d'acétyl-L-carnitine et de propionyl-L-carnitine dans un échantillon, qui comprend :
(a) soumission d'une partie de l'échantillon à une hydrolyse enzymatique avec une acylcarnitine estérase qui n'a pas d'activité enzymatique pour l'acétyl-L-carnitine et la propionyl L-carnitine mais qui montre une spécifité pour le substrat aux acyl-L-carnitines de longueur de chaîne plus longue et qui catalyse la réaction d'hydrolyse d'une mole d'acyl-L-carnitine desdites longueurs de chaînes plus longues avec une mole d'eau pour former une mole de l'acide gras correspondant et une mole de L-carnitine et ensuite détermination de la quantité des acides gras et/ou de la L-carnitine ainsi formée ;
(b) soumission d'une autre partie de l'échantillon à un dosage des acyl-L-carnitine incluant l'acétyl-L-carnitine et la propionyl-L-carnitine telle que définie dans l'une quelconque des revendications 5 à 8 ;
(c) détermination de la différence des valeurs analytiques obtenues aux étapes (a) et (b).

10. Méthode selon la revendication 9, dans laquelle l'acylcarnitine estérase qui n'a pas d'activité enzymatique pour l'acétyl-L-carnitine et la propionyl-L-carnitine mais qui montre une spécificité pour le substrat aux acyl-L-carnitine de longueurs de chaînes plus longues a été obtenue d'un rat.

11. Méthode de dosage d'acyl-L-carnitine et de propionyl-L-carnitine dans un échantillon, qui comprend :
(a) la soumission de l'échantillon à un hydrolyse enzymatique avec une acylcarnitine estérase qui n'a pas d'activité enzymatique pour l'acétyl-L-carnitine et la propionyl-L-carnitine mais qui montre une spécificité de substrat pour les acyl-L-carnitines de longueurs de chaînes plus longues et qui catalyse la réaction d'hydrolyse d'une mole d'acyl-L-carnitine de longueurs de chaînes plus longues avec une mole d'eau pour former une mole de l'acide gras correspondant et une mole de L-carnitine et ensuite soumission du mélange de l'échantillon réagi résultant à l'action d'une L-carnitine déhydrogénase avec un coenzyme A₁ (où A₁ est un composé du groupe des NAD ou des thio-NAD) pour convertir A₁ en sa forme A₂ réduite, et le traitement du mélange échantillon résultant pour des composés A₂ ; et
(b₁) soumission du mélange échantillon résultant à une méthode de dosage des acyl-L-carnitines incluant l'acétyl-L-carnitine et la propionyl-L-carnitine telle que définie dans l'une quelconque des revendications 5 à 8.

12. Alcaligenes sp. N° 981 (FERM BP-2570) ou un mutant de celui-ci qui est capable de produire une acylcarnitine estérase telle que définie en revendication 1.

13. Culture d'Alcaligenes sp. N° 981 (FERM BP-2570) ou de son mutant qui est capable de produire une acylcarnitine estérase telle que définie en revendication 1, dans un milieu de culture qui comprend une source de carbone assimilable, une source d'azote assimilable et des sels minéraux et qui est exempte d'autres microorganismes.
